# EUROPEAN PATENT SPECIFICATION

(11) **EP 3 237 038 B1**
(45) Date of publication and mention of the grant of the patent: **08.04.2020**
(21) Application number: 15874126.4
(22) Date of filing: 14.12.2015
(51) Int. Cl.: A61M 3/02, A61M 11/06, A61M 15/08

(54) **PORTABLE FLUID DELIVERY SYSTEM FOR THE NASAL AND PARANASAL SINUS CAVITIES**
TRAGBARES FLÜSSIGKEITSABGABESYSTEM FÜR NASEN- UND NASENNEBENHÖHLE
SYSTÈME DE DISTRIBUTION DE FLUIDE PORTABLE POUR LES CAVITÉS DES SINUS NASAUX ET PARANASAUX

(30) Priority: 22.12.2014 US 201414579270
(43) Date of publication of application: 01.11.2017
(73) Proprietor: NasoNeb, Inc., Medina, OH 44256 (US)
(72) Inventor: FLICKINGER, William J., Medina, OH 44256 (US)
(74) Representative: Maldonado Jordan, Julia
(86) International application number: PCT/US2015/065442
(87) International publication number: WO 2016/105985

(56) References cited:
- US-A1- 2005 048 436
- US-A1- 2009 247 941
- US-A1- 2011 040 250
- US-A1- 2011 040 250
- US-A1- 2011 132 354
- US-A1- 2012 160 237
- US-A1- 2014 171 880
- US-A1- 2014 283 820
- US-B1- 6 732 731

## Description

### TECHNICAL FIELD

The present invention generally relates to devices used for administering fluid to the upper airway in mist or droplet form, either for the irrigation of the nasal passages or the delivery of medication.

### BACKGROUND OF THE INVENTION

Devices used for administering liquid medication to a patient by way of mist or liquid droplets are generally called nebulizers and are primarily used for the delivery of medication into the lungs. These devices are best suited for the inhalation of the mist or aerosol through the patient's mouth. However, some cases require the introduction of liquid droplets to the patient's nasal passages and the droplet or particles of the aerosol generated by such nebulizers are small and lightweight so as to pass through the nasal cavity to the lungs on the inhaled air leaving very little if any of the aerosol deposited in the nasal cavity.

Another device used for delivering liquid through a patient's nasal passages is a nasal irrigator, or irrigator. Irrigators, however, deliberately deliver liquid to the patient's nasal passages, rather than bypassing the nasal cavity to reach the lungs. Current irrigators for introduction of medication to or irrigation of the nasal passages generally comprise an air compressor, an irrigator cup for the liquid medication, and compressor tubing to connect the compressor to the irrigator cup. Inevitably, the equipment and parts used with current irrigators are large and extremely bulky, weighing at least ten pounds. Thus, the compressor tubing provides for a convenient way of handling the irrigator cup during irrigation or use; however, the compressor itself is not a portable or lightweight device. To use the irrigator, the compressor must be placed on a sturdy surface in order to support its weight and its power supply cord must be plugged into an outlet.

Document US 2012160237 A1 discloses a nasal nebulizer with a main canister for receiving fluid and an insert with a fluid channel including a contiguous pressurized air supply source. The fluid channel is a tapered tube overlapping an air exit port in the canister. The fluid channel comprises a common bell housing surrounding the air exit port above a base of the insert, which rests within the canister. The insert includes an extension projecting out to the canister and a vertical groove extends down the fluid channel to a hole in the extension, which upon use helps to create a vacuum that pulls in deflected fluid. The main canister includes a convex top surface around the periphery of an opening for a reservoir that receives fluids. The top surface extends downwardly to a generally rectangular opening that connects to the pressurized air supply source.

Document US 2011040250 A1 discloses a device for washing nasal cavities by a nebulized liquid, that comprises a main body, a tank for liquids including at least a nebulization chamber, and means of collection and nebulization of the treatment liquid from said nebulization chamber and delivery of the nebulized liquid towards the nasal cavities with the help of a flow of air under pressure generated by a compressor group. The main body has a cavity and the compressor group, a piloting electronic circuit and an electric battery system are all on board of a single support housed and retained in the cavity of said main body.

### SUMMARY OF THE INVENTION

The invention is defined in the appended claims. Where in the following the word invention is used and/or features are presented as optional this should be interpreted in such a way that protection is sought only for the invention as claimed.

The components of an irrigator comprise a size and weight that simply do not allow for convenient transport, portability or even quick and simple handheld usage. There is a need for an irrigator device that will easily fit in well with a person's daily needs or activities such that the person need not have to change his or her daily routines or schedule times of day to be close to an irrigator or an outlet for the irrigator. The device should be easy to carry on one's person and provide convenience to the needs of a patient. There is a need for an all-in-one irrigator device that contains all the components necessary to successfully use an irrigator, without the heavy weight of its components and without tubing connected to heavy equipment, and without compromising the performance needed to penetrate the nasal and paranasal cavities. There is also the need to eliminate the requirement to be close to a power outlet to be able to use an irrigator.

A portable, ready-to-use device for nasal irrigation and/or drug delivery of fluid is provided herein, which allows for more convenient use, not requiring connecting tubing, a power supply cord, or a heavy or bulky compressor. The irrigator device has an internal airflow regulating system within a connected pressurized air supply source with airflow regulating components that replace heavy, large components to power atomization of fluid for irrigation or drug delivery to the nasal passages without compromising effectiveness or reach of the atomized fluid. The airflow regulating system comprises an air outlet, a pump, a motor, a filter for filtering incoming air, and a circuit board to control the motor. The pump is in communication with the main canister and the motor, and the filter connects to the pump. The motor is driven by one of: a battery contained within the pressurized air supply source or an external power supply source. A canister for holding fluid attaches on top of the pressurized air supply source and an insert fits that over a fluid channel in the canister to create a venturi effect and draw the fluid out. The entire device described herein, as a whole, (meaning all its parts and components) is lightweight (i.e., less than 2 pounds) and easy to handle and hold either single-handedly or with both hands; thereby, cutting down the weight and bulky equipment otherwise necessary by more than half. Where the device is used without an external power supply, the lightweight device weighs only about one pound. With an external power supply, the portable device described herein weight about 1.5 lbs. In addition, the device is small enough to be carried or moved with ease to provide for convenience while still having the power to reach the desired areas of the nasal passage and the particle size to minimize or eliminate the risk of pulmonary delivery and keep the aerosol in the nasal cavity. The device can also be readily used at any time without having to search for a nearby electrical outlet, when a battery pack within the pressurized air supply source has sufficient charge.

The fluid to be administered to a user is contained within a canister of the device. The canister is recessed within a concave top portion of the handheld pressurized air supply source comprising the airflow regulating system therein. A lip of the canister attaches on top of the rim of the pressurized air supply source. An insert with a tapered fluid channel fits over tubular channel or tube having an air exit port at its top end. The insert includes an extension projecting out to the canister from the fluid channel. A vertical groove may extend down the exterior of the fluid channel to a hole in the extension that allows for venting of air or spent fluid into the enclosed canister as the fluid to be delivered is displaced during use. The fluid is atomized via the attached airflow regulating system to create particles sized for dispersion and retention within the nasal cavity delivered via a pressurized flow that is able to drive the aerosol past structures in the nasal cavity that act to filter the inhaled airstream to deliver the resultant mist into the whole of the nasal passages without the need for the patient to create an airstream through inhalation.

As stated above, the device requires no connecting tubing and no power cord during use to power the atomization of fluid. Instead, the airflow regulating system within the pressurized air supply source comprises substantially all or all components necessary to regulate and control the air supply. The pressurized air supply source houses a motor that drives a pump, which receives filtered air through a filter on the pressurized air supply source, and an optional rechargeable battery to drive the motor. An inlet air manifold of the filter connects to the pump via a pump air inlet post to eliminate additional tubing within the pressurized air supply source. A circuit board precisely controls the motor speed to ensure the proper airflow, ensures the battery voltage is maintained at a proper voltage for operation and drives indicators to inform the user when the battery requires charging and is being charged. The AC/DC power supply charges the battery and provides the user the option to operate the device on mains power when the battery is discharged. The motor controller board may utilize pulse width modulation or alternatively digital control to control motor speed within a narrow band to regulate airflow generated by the pump.

A single membrane on one external side of the pressurized air supply source contains substantially all the electrical components externalized to the user and incorporates a single ribbon connector. A power jack is the only electrical component outside the single membrane. A tethered cover may be used to cover the power jack and reduce fluid and dust ingress to the device when the power supply is not plugged into the irrigator, such as when the pressurized air supply source houses a battery. When pressurized air is introduced through the air inlet of the canister, a venturi effect is created, drawing fluid up between the air exit port and fluid channel and expelling the fluid as a mist through a discharge port in the fluid channel. The mist may comprise medication, saline or other non-active ingredients to provide moisture.

Other aspects, embodiments and features of the invention will become apparent from the following detailed description of the invention when considered in conjunction with the accompanying drawings. The accompanying drawings are schematic and not intended to be drawn to scale. In the figures, each identical or substantially similar component that is illustrated in various figures is represented by a single numeral or notation. For purposes of clarity, not every component is labeled in every figure. Nor is every component of each embodiment of the invention shown where illustration is not necessary to allow those of ordinary skill in the art to understand the invention.

### BRIEF DESCRIPTION OF THE DRAWINGS

The novel features believed characteristic of the invention are set forth in the appended claims. The invention itself, however, as well as mode of use and advantages thereof, will best be understood by reference to the following detailed description of illustrative embodiments when read in conjunction with the accompanying drawings, wherein:
**Figure 1** is an exploded view of a nasal irrigator in accordance with an embodiment of the present invention;
**Figure 2** shows a cross sectional view of a canister in accordance with an alternate embodiment of the invention;
**Figure 3** shows an alternate embodiment of the cover in accordance with the present invention;
**Figure 4** illustrates the use of the nasal irrigator of **Figure 1** in accordance with an embodiment of the present invention;
**Figure 5** conceptually illustrates the function of the nasal valve in aerosol delivery that is initiated below the nasal valve;
**Figure 6** shows an embodiment of a nasal irrigator in accordance with an embodiment of the present invention;
**Figure 7** is a schematic cross sectional view of the assembled nasal irrigator of **Figure 6**;
**Figure 8** shows a perspective view of an assembled nasal irrigator in accordance with an embodiment of the present invention;
**Figure 9a** shows an exploded view of an embodiment of a nasal irrigator in accordance with an embodiment of the present invention.
**Figure 9b** shows a bottom view of an insert in accordance with an embodiment of the present invention.
**Figure 10** shows a perspective view of an assembled nasal irrigator in accordance with an embodiment of the present invention.
**Figure 11** is a schematic cross sectional view of the assembled nasal irrigator of **Figure 10**;
**Figure 12a** shows an exploded view of a nasal irrigator in accordance with an embodiment of the present invention.
**Figure 12b** shows a bottom view of an insert in accordance with an embodiment of the present invention.
**Figure 13a** shows a top perspective exploded view of the nasal irrigator of **Figure 12**.
**Figure 13b** shows a cross-sectional side view of an assembled irrigator in accordance with the present invention;
**Figure 14** shows a perspective view of an assembled nasal irrigator in accordance with the present invention;
**Figure 15** shows an exploded view of a nasal irrigator in accordance with an embodiment of the present invention.
**Figure 16** shows a perspective view of an assembled nasal irrigator in accordance with an embodiment of the present invention;
**Figure 17** shows a top view of the nasal irrigator of **Figure 16****.**
**Figure 18** shows a schematic cross sectional view of a filter in accordance with an embodiment of the present invention.
**Figure 19A** shows an exploded view of a portable irrigator according to an embodiment of the present invention.
**Figure 19B** shows another perspective view of the portable irrigator shown in **Figure 19A****.**
**Figure 20** shows a front perspective view of an assembled portable irrigator as shown in **Figures 19A** and **19B****.**
**Figure 21A** shows a perspective view of an assembled portable irrigator according to an embodiment of the present invention.
**Figure 21B** shows a perspective view of a portable irrigator as depicted in **Figure 21A**.
**Figure 22** shows a cross sectional detailed view of a portion of the main canister of an assembled portable irrigator according to an embodiment of the present invention.
**Figure 23** shows a perspective view of an assembled portable irrigator according to an alternate embodiment of the present invention.
**Figure 24** shows an exploded view of another embodiment of a portable irrigator.
**Figure 25** shows a perspective view of a portable irrigator with the canister attached to the pressurized air supply source.
**Figure 26A** is a top view of the pressurized air supply source in one embodiment.
**Figure 26B** is a partial cross-sectional view of the canister attached to the pressurized air supply source.
**Figure 27** shows a perspective view of a portable irrigator with the insert attached to the canister and pressurized air supply source.
**Figure 28** shows a cross-sectional view of the insert in one embodiment.
**Figure 29** depicts an assembled view of one embodiment of the portable irrigator with a cap over the insert.
**Figure 30** shows a side perspective view of a partial cross-section of the canister attached to the pressurized air supply source.
**Figure 31** shows a view of the components within the pressurized air supply source of a nasal irrigator in one embodiment
**Figure 32** shows a cross-sectional view of the nasal irrigator of Figure 31.
**Figure 33A** shows a partial view of one embodiment of the assembled irrigator with one side of the pressurized air supply source removed.
**Figure 33B** shows a partial view of one embodiment of the assembled irrigator with the other side of the pressurized air supply source removed.
**Figure 34A** shows a perspective view of an inlet air manifold in one embodiment.
**Figure 34B** shows another perspective view of an inlet air manifold in one embodiment.
**Figure 35** shows a perspective view of a filter cap of the portable irrigator in one embodiment.

### DETAILED DESCRIPTION

The present invention improves upon current irrigator designs and provides a method of delivering fluid to the nasal passages with little interaction required by the user, under sufficient pressure to stent-open the airway, and with particles of a size to ensure that the majority of the mist is retained or deposited within the upper airway. The invention also provides a nasal irrigator designed to deliver a mist to the upper airway through both nostrils simultaneously.

In one aspect, a nasal irrigator of the present invention comprises a main canister with a reservoir for holding fluid, wherein the canister includes at least two air exit ports; a removable insert with a circular base that fits within said main canister, wherein the insert includes at least two fluid channels that mate with said air exit ports of the main canister, said fluid channels comprising two tubes ending in a common bell housing above the base, wherein said base holds the insert just off of the main canister surface, allowing fluid to pass between the base and main canister, and further wherein the fluid channels are larger in diameter than the air exit ports, thereby providing a small space between the outer surface of the air exit ports and the inner surface of the fluid channels that allows fluid from said reservoir to be drawn upward between the air exit ports and fluid channels and expelled as a mist in an aerosol plume through exit holes in the fluid channels due to a venturi effect created by pressurized air from the air exit ports; and at least one nozzle coupled to the bottom of said main canister to create at least one air chamber defined by the nozzle and said air exit ports, wherein the nozzle includes an air inlet for providing pressurized air into said air chamber.

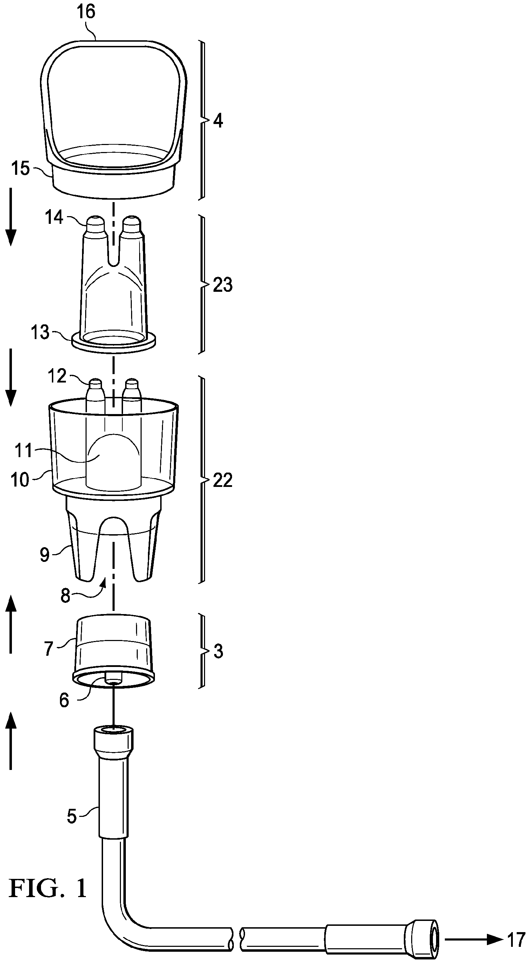

**Figure 1** is an exploded view of a nasal irrigator in accordance with an embodiment of the present invention. The nasal irrigation device comprises three major sections. The first major section is the main canister **22** which has an expanded reservoir **10** that is capable of holding up to 50 ml of fluid. The inner portion of the reservoir shaped at the bottom to ensure maximal uptake of fluid to reduce waste.

The main canister **22** also includes an air chamber **11** terminating in two air exits **12** (one for each nostril) with holes sufficient to deliver an airstream that is able to atomize fluid and stent-open the upper airway. In one embodiment, each exit port **12** has at least one hole of between 0.020" and 0.060" (0.508 mm - 1.524 mm) in diameter and a web-thickness or hole length of between 0.030" and 0.200" (0.762 mm - 5.08 mm).

On the bottom of the main canister **22** is a foot section **9** that includes one or more feet for stability and an air inlet **8** for the admission of pressurized air to create the air stream through air exits **12**. The foot section **9** enables the canister **22** to stand up when set on a horizontal surface and is designed to fit into a standard docking port of an air compressor pump to enable the device to remain upright in a hands-free manner so as to remain filled with the air supply tube attached.

In the shown example, the main canister **22** has a two-step circumference to fit a holder (not shown) and provide adequate fluid volume for nasal irrigation, with the smaller diameter foot section **9** enabling the user to rest device in the holder with tube attached. In an alternate embodiment (not shown) the foot section **9** is wider than the reservoir section **10**.

The second major section of the irrigator is the insert **23**, which is shown with a base **13** that holds the inside surface of the insert **23** just off of the outer surface of the feature within reservoir **10** of the main canister **22**. At least one channel is located in the bottom of the insert **23** to act as a conduit for fluid from the reservoir **10** to enter the base of the insert. The insert **23** includes fluid channels **14** that mate with the air exit ports **12** of the main canister **22**. Peaks or extensions may be included on the air exits **12** to ensure centering of the insert **23** and its fluid channels **14** on the air exits. Similarly, tabs may extend from the inside of the fluid channels of the insert to the outer surface of the main canister to ensure alignment. As shown, fluid channels **14** of the insert **23** comprise two tubes with one end at the bottom of the reservoir **10** and one end that is positioned in the airstream so that the airstream creates a negative pressure in each tube that draws fluid into the airstream where it is atomized (described below).

In the embodiment shown in **Figure 1**, the atomizer outlets **12**, **14** extend above the edge of the main canister **22**. However, in an alternate embodiment (not shown) the atomizer nozzles are even with or recessed within the edge or portions of the edge of the main canister.

The insert **23** is keyed in at least one location with the reservoir **10** to ensure that the insert does not rotate in relation to the exit ports **12** of the main canister and to aid in centering of the insert **23** and its fluid channels **14** on the air exits. The insert may include a feature to ensure that it is inserted into the main canister in only one orientation. In one embodiment, a loop (not shown) extends down to the saddle of the insert **23** to hold down the insert.

The fluid channels **14** are slightly larger in diameter than the air exit ports **12** of the main canister, thereby providing a small space (preferably 0.0001" to 0.010" (0.00254 - 0.254 mm)) between the outer surface of the air exit ports and the inner surface of the fluid channels. This space allows fluid from the reservoir **10** to proceed upward between the air exit ports **12** and the fluid channels **14** until being expelled by pressurized air. When the insert **23** is installed in the main canister **22**, the orifices of the fluid channels **14** are positioned relative to the air exits **12** so as to create a venturi effect with the pressurized gas expelled from the gas tubes. Because the fluid exits **14** in the insert **23** are larger than the air exits **12**, when air is forced through the air exits at an appropriate volume and speed, fluid in the reservoir **10** is drawn up into the space between the insert and air exits ports. When this fluid meets the subsequent airstream it is atomized into particles conducive to deposition in the upper airway. The airstream is sufficient to penetrate the nasal cavity above the inferior turbinate so as to deposit the fluid and provide a washing, irrigation, or deposition to the upper reaches the nasal cavity.

The exit holes of the fluid channels **14** are small enough to ensure that mist is created but large enough to ensure that the holes of the insert may be chamfered so that the walls of the exit holes are angled away from a central axis at an angle that exceeds the cone of the aerosol plume to reduce agglomeration of the mist particles upon exit, providing a more uniform particle size throughout the plume. The fluid channel size may be adjusted to change the particle size of the mist. In one embodiment the tubes have a mating section on the upper end that enables the changing of the orifice in the air stream via a series of nozzles that can be inserted into the upper end of the tubes such that the size of the nozzle orifice that is placed into the airstream is varied.

The third major section of the irrigator is nozzle cone **3**. The nozzle **3** includes an air inlet **6** and a mating surface **7**, which attaches to the air inlet **8** of the main canister **22** to create air chamber **11** defined by the nozzle and the two exit ports **12** described above. The length of all components on the nozzle cone **3** preferably is limited so that the nozzle cone or its components do not extend past the foot section **9** on the main canister **22** when the device is assembled to enable the device to be placed on a flat surface in an upright or standing position.

Ribs may also be molded into the nozzle cone **3** to provide radial stiffness. In another embodiment, the nozzle cone is made of rigid plastic.

The mating surface between the nozzle **3** and main canister **22** is designed to ensure a tight bond can be created. In an alternate embodiment the mating surface between the nozzle **3** and main canister **22** is essentially straight.

In one embodiment, the nozzle cone **3** is attached permanently to the main canister **22**. In an alternate embodiment, the nozzle cone **3** may utilize a friction fit or have a positive connection such as a thread or other mechanism allowing the nozzle cone and main canister **22** to be disconnected for cleaning. This detachable embodiment may include an air seal such as an O-ring as well as a flange to grasp for easy disassembly.

An air supply tube **5** connects the air inlet **6** of the nozzle cone with an air supply **17**.

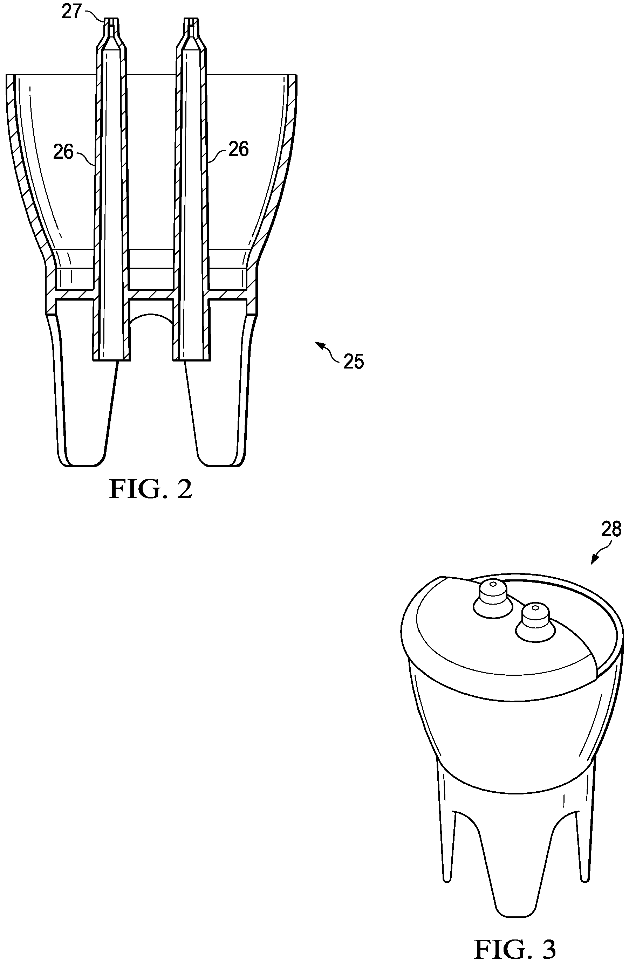

**Figure 2** shows a cross section view of a canister **25** in accordance with an alternate embodiment of the invention. In this embodiment, rather than a single air chamber and nozzle, the canister **25** includes separate air passage chambers **26** that terminate in the air exits **27**. These separate air passage chambers **26** can connect to separate air sources via separate nozzles. Alternatively, the separate air passage chambers **26** can be connected to a common air source via split tubing such as a Y or T adapter (not shown).

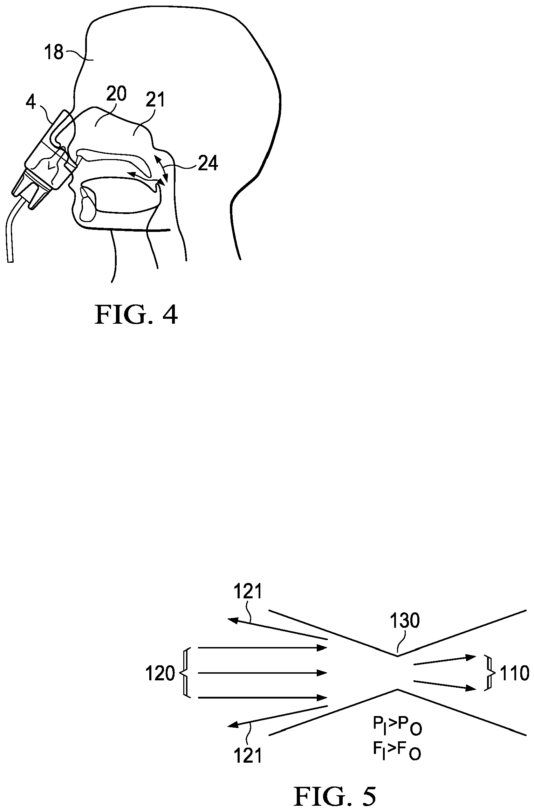

In addition to the three major sections described above, the irrigator may include a cover **4** that has a mating surface **15** that creates an isodiametric connection to the main canister **22**. In the example shown in **Figure 1**, the cover **4** is a broad cover region to block space between the nose, eyes and the rest of the face when in use as shown (see **Figure 4**). In this embodiment the cover **4** is designed to confine the mist expelled from the fluid channels and shield the patient's eyes, with an opening to provide room for the patient's nose within the apparatus. The cover **4** is radiused along the distal end away from the main canister **22** to fit a broad variety of faces and is open to enable air to enter as the fluid is drawn down and capture and recycle fluid that falls off the face.

The cover may also incorporate a cross member or other device that retains the insert **23** to allow for clearance of the nose and prevent lifting of the insert at the initiation of atomization. In one embodiment a sleeve or partial sleeve extends from the cover **4** to the base of the insert **23** to hold the insert down.

**Figure 3** shows an alternate embodiment of the cover in accordance with the present invention. In this embodiment, the cover **28** is a semi-circular lid that does not block the eyes but instead retains the insert and blocks material from re-entering the main canister from the nose.

The present invention may incorporate a feature that guides the user to angle the spray into the nose at a set angle from 0 - 90 degrees from the plane defined as the front of the face from the chin to the forehead (i.e. the vertical plane of the face). For example, the irrigator may include a setoff designed to set a specific angle of 30 degrees, 45 degrees, or 60 degrees from the vertical plane of the face. The setoff may be removable for various size faces or noses.

Materials suitable for construction of the irrigator include rigid plastic, glass, metal, ceramic, carbon fiber or other rigid material, or an elastomer plastic or some combination thereof.

One embodiment of the nasal irrigation device (not shown) is egg-shaped or ovoid for better fit into the hand and a pleasing look.

**Figure 4** illustrates the use of the nasal irrigator in accordance with the present invention. The irrigator is placed over the face of the user **18** and angled such that the cover **4** blocks the eyes. The mist **20** enters the nasal passages **21**, and the patient breathes through both the mouth and nose at the same time (**24**). The mist **20** passes into the nasal passages **21** independent of the patient's breathing.

The air-fluid mixture is calibrated to achieve nasal irrigation within a short period of time, without the need for the fluid to exit the nostrils at the time of irrigation, and with a particle size that is designed to loosen the mucous or to enter the sinus cavities, as desired by the end user and not enter the pharynx or the lungs.

In one aspect, the method of nasal irrigation comprises providing fluid in a canister that includes at least two air exit ports mated to corresponding fluid channels, wherein the fluid channels are larger in diameter than the air exit ports, thereby providing a small space between the outer surface of the air exit ports and the inner surface of the fluid channels. This space allows fluid from said reservoir to be drawn upward between the air exit ports and fluid channels. Pressurized air is pumped through the air exit ports, thereby creating a venturi effect that draws fluid from said reservoir upward between the air exit ports and fluid channels and expels the fluid as a mist in an aerosol plume through exit holes in the fluid channels and into a user's nasal cavity above the inferior nasal turbinate independent of the user's breathing. The pressurized air has a pressure of 0.069 - 1.035 bar and an airflow rate of 1 - 12 liters per minute, producing a fluid delivery rate of 1 - 20 ml per minute.

The method of nasal irrigation offers a fast, convenient method of atomizing saline or medication for delivery to the nose, with a variable particle size up to 100 microns. In one embodiment, particle size is at least 10 microns.

Using an air pressure of 1 - 15psi (0.069 - 1.035bar) creates a pressurized airflow that enables the resultant air-mist stream to stent-open the soft tissues of the upper airway. In one embodiment, the air pressure ranges from about 3 - 12psi (0.207 - 0.823bar), with about 1 - 12 lpm of airflow, and a fluid delivery rate of about 1 - 20ml per minute. In one embodiment, the air pressure ranges from about 4 - 8psi (0.276 - 0.552 bar), with about 3.5 - 8 lpm airflow, and about 15ml per minute fluid delivery.

The resultant mist reaches the area of the nasal cavity and paranasal sinuses above the inferior nasal turbinate or chonchae to ensure that the mist reaches the areas of the sinus ostia to clear this area of the nasal cavity and enable the natural mucociliary flow to clear the sinuses.

Recent medical research has noted that the olfactory and trigeminal nerves may be used as a pathway to deliver large and small molecules to the brain and central nervous system that bypasses the blood brain barrier and first pass metabolism of intravenous and oral delivery routes. (See Dhanda, D., Frey WH 2nd, Leopold, D., Kompella, UB: "Nose-to-brain delivery approaches for drug deposition in the human olfactory epithelium." Drug Delivery Technol. 5(4), 64-72 (2005).) Frey and others have demonstrated that these nerves may be reached via the nasal mucosa overlying the olfactory cleft and cribriform plate where these nerves are concentrated. Furthermore, the frequency of dosing of many of these materials requires a delivery system that is practical and easy to use. In the case where systemic delivery of drugs via the nose is desired, maximizing the surface area of the mucosa covered by the medication may improve the amount of medication that is absorbed by the body and may reduce the variability of absorption between doses and across patients; thus improving the bioavailability of the drug and reducing the variability of bioavailability of the drug. Furthermore, by maximizing the surface area available for absorption of any given drug, the concentration required to deliver an effective dose may be reduced when compared to traditional metered dose inhaler technology, enabling more drugs to be delivered transnasally than with other systems.

However, the literature suggests that adequate delivery systems are lacking for the reliable and practical delivery of these substances to these areas. Delivery of large particles (>10 microns) of liquids in the described volumes as provided by the present invention, offers advantages over dry powder, minute volumes and high volume solutions. These advantages include covering the whole nasal mucosa, formulating drugs for patient comfort vs. concentration, reducing the inadvertent delivery of aerosolized materials to the lungs; and the ability to deliver precious materials economically and judiciously while reducing waste.

In one aspect, the present invention provides a method of treating neoplasms of the nasal cavity comprising fluid in a canister, wherein the canister includes a reservoir and at least two air exit ports, and wherein said fluid contains corticosteroids. The air exit ports are mated to corresponding fluid channels, wherein the fluid channels are larger in diameter than the air exit ports, thereby providing a space between the outer surface of the air exit ports and the inner surface of the fluid channels, which allows fluid from said reservoir to be drawn upward between the air exit ports and fluid channels. Pressurized air is pumped through the air exit ports, thereby creating a venturi effect that draws fluid from said reservoir upward between the air exit ports and fluid channels and expels the fluid as a mist in an aerosol plume through exit holes in the fluid channels and into a user's nasal cavity above the inferior nasal turbinate independent of the user's breathing.

The present invention allows for delivering steroids for the long-term control of benign neoplasms of the nasal cavity, such as inflammatory nasal polyps, granulomas, etc., without systemic doses of steroids or steroid injections. It also provides the ability to irrigate the whole nasal mucosa to manage the disruption of natural filtering and humidification often caused by ablative and reconstructive surgical treatment of neoplasms. Unlike prior art saline irrigation and nasal sprays which do not reach many of the areas of concern in the nasal vestibule and paranasal sinus areas, the irrigator of the present invention delivers adequate moisture in less than one minute to the areas of concern. The present invention also avoids pooling of moisture that can otherwise provide a nidus for infection and cause excessive removal of the immunologic mucus blanket of the nose.

The high frequency of steroid administration needed to control neoplasm growth requires a delivery system that is practical and easy to use. The irrigator of the present invention can deliver these steroids quickly - in less than one minute - covering the whole nasal cavity and does so without unduly exposing the body to the effects of systemic steroids.

For example, using the irrigator of the present invention, 0.60 mgs of corticosteroid is typically delivered to the nasal cavity, between two and ten times the amount delivered via metered dose inhalers. In some instances, antibiotics are delivered along with the corticosteroid to treat infections such as Staphylococcus aureus. Staph aureus endotoxin has been shown to up-regulate the beta isoform of cortisol receptor (CR_{β}) in cell membranes that is responsible for inhibiting the response to corticosteroids, and it is believed that the Staph infection may contribute to steroid-resistant nasal polyps. The concurrent administration of antibiotics with the corticosteroid via the irrigator of the present invention reduces this endotoxin effect on the cortisol receptor, thereby increasing the efficacy of the steroid therapy.

The pressure and airflow necessary to deliver material to the upper portion of the nose can be reduced if the aerosol is introduced distal of the nares at or above the nasal valve and proximal to the inferior turbinate. The present invention delivers droplets or mists with an air stream and particle sizes designed to stay in the upper airway under sufficient pressure and airflow to overcome the normal aerodynamics of the nose. Unlike prior art methods, the present invention releases mist at or above the nasal valve, thereby avoiding deflection of the fluid off the walls of the nostril and nasal valve.

Effective delivery of material to the nasal cavity requires a particle size that is large enough to fall out of the airway before reaching the oropharynx, delivered under sufficient pressure and airflow to overcome the aerodynamics of the nasal cavity. The nasal cavity is shaped to efficiently deliver air to the lungs. Air enters the nares and passes through the nasal valve, which resides approximately 1.3 cm above the nares and is the narrowest portion of the nose, with a cross-section of at approximately 0.73 cm². The nasal valve is the narrowest anatomic portion of the upper airway, resulting in the volume of air inspired nasally to be efficiently cleansed and humidified by the nasal cavity.

**Figure 5** conceptually illustrates the function of the nasal valve in aerosol delivery that is initiated below the nasal valve. Arrows **120** represent an aerosol flowing into the nasal nares. As illustrated by arrows **121**, a portion of this aerosol is reflected off the walls of the nose as the passageway narrows to the nasal valve **130**. This reflected material falls out of the nose and is either wasted or is recollected by the device to be delivered repeatedly.

The nasal valve **130** acts to reduce the flow (F) and pressure (P) of that portion of the aerosol stream that crosses the valve and enters the nasal cavity **110**. Thus, Flow in (F_{I}) is greater than Flow out (F_{O}), and Pressure in (P_{I}) is greater than Pressure out (P_{O}). As a result, aerosol entering the nasal cavity external to the nasal valve requires a higher pressure and flow rate to achieve the same aerosol distribution as an aerosol introduced internal to the nasal valve.

Air entering the nose meets additional resistance at the level of the inferior turbinate, which directs air downward along the floor of the nose along the path of least resistance. During inhalation, the airflow is dominated by the negative pressure being generated from the lower airway and is directed to the nose from the pharynx. This negative pressure and the structure of the nasal cavity conspire to direct the majority of the air through the lower third of the nose, with very little air entering the upper portion of the nose. Indeed, studies have shown that to reach the upper portion of the nose under the negative pressure of normal breathing, an aerosol must be placed very precisely at the front of the nares. To overcome the aerodynamics of the nose, the delivery system must provide a positive pressure and sufficient airflow to fill the whole nasal cavity.

Prior art devices that deliver aerosol below the nasal valve must generate higher pressure and flow rates since the valve acts to lower the pressure and flow as the aerosol passes through it. The design of the present invention is directed to the self-administration of fluid to the nasal passages of a patient while ensuring the device fits a wide variety of faces and for simplicity of design, ease of manufacturer. It requires lower pressure and airflow and produces less mess by virtue of delivery above the nasal valve, and simplicity of use, including short delivery times.

The invention delivers fluid to the nasal passages with little interaction required by the user and under sufficient pressure to stent-open the airway. The invention delivers particles of a size to ensure that the majority of the mist is retained or deposited within the upper airway, while maximizing the amount of drug delivered and eliminating reflection back from the nasal valve.

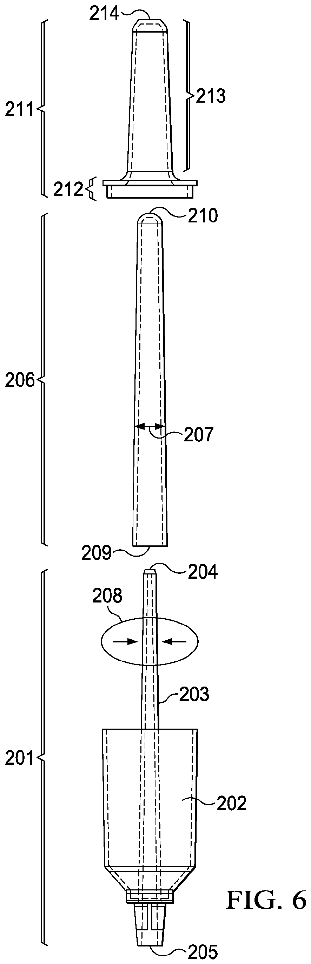

**Figure 6** shows an embodiment of a nasal irrigator in accordance with the present invention. The nasal irrigator comprises three main components. The first component is the main canister **201**, which has a fluid reservoir **202** and an air exit port **203** that extends above the reservoir. In one embodiment, the reservoir **202** holds up to 30 ml of fluid or medication. As shown in Figure 1, the lower portion of the reservoir is downward sloping to ensure fluid collects at the bottom, which allows maximal uptake of fluid through fluid channels (explained below), thereby minimizing waste.

The air exit port **203** has at least one exit hole **204** at the top sufficient to deliver an airstream that is able to atomize fluid and deliver the aerosol to the whole nasal cavity. In one embodiment, the exit hole **204** is between 0.020" (0.508mm) and 0.060" (1.524mm) in diameter and the air exit port has a web-thickness of between 0.030" and 0.200" (0.762 mm - 5.08 mm).

The main canister **201** also included an air inlet **205** on the bottom for the admission of pressurized air to create the air stream exiting the air exit port **203**.

In one embodiment, the main canister **201** has optional "feet" on the bottom (as shown in **Figure 1**) for stability. The length of all components on the nozzle cone is limited so that the nozzle cone or its components do not extend past the feet on the main canister when the device is assembled to enable the device to be placed on a flat surface in an upright or standing position. The canister **201** may also be designed to fit into a standard docking port of an air compressor to enable the device to remain upright in a hands-free situation so as to be filled with the air supply tube attached.

The second main component of the nasal irrigator is an insert **206** that fits over the main canister's air exit port **203**. The insert **206** can be permanently attached to the canister **201** or it may be removable. The insert **206** has an aerosol exit **210** that is concentrically aligned with the exit hole **204** of the air outlet **203**. A peak or extension on the air exit port **203** may ensure centering of the insert over the air outlet. Similarly, tabs on the insert may be used to center the insert over the air outlet and prevent it from being moved by force. The aerosol exit **210** is slightly larger than the exit hole **204** of the air exit port **203** to enable atomization of fluid in the air stream.

The insert **206** has a tapered inner diameter **207** that is larger than and follows the contours of the outer diameter **208** of the air exit port **203**. This difference in diameter creates a space of between 0.0001" (0.00254mm) and 0.010" (0.254mm) between the inner surface of the insert **206** and the outer surface of the air exit port **203**. This space allows fluid to be drawn from the reservoir **202** through a channel **209** at the base that is sized to control the fluid flow.

The third main component of the nasal irrigator is the cover **211** that mates with the reservoir **202** of the main canister **201** and extends over the insert **206** such that the insert does not contact the nose as the device is inserted into the nasal cavity, thereby ensuring that the hole **210** in the insert **206** and the hole **204** in the air exit port **203** remain concentrically aligned. The cover **211** includes a mating surface **212** that creates a preferably isodiametric connection to the main canister **201** and extends around the nozzle formed by the insert **206** and air exit port **203**. The cover **211** extends just above the insert **206** and has its own exit hole **214** designed not to restrict the flow of the aerosol plume. In one embodiment, the cover **211** provides a cross member or other feature that secures the insert **206** to prevent lifting of the insert at the initiation of atomization.

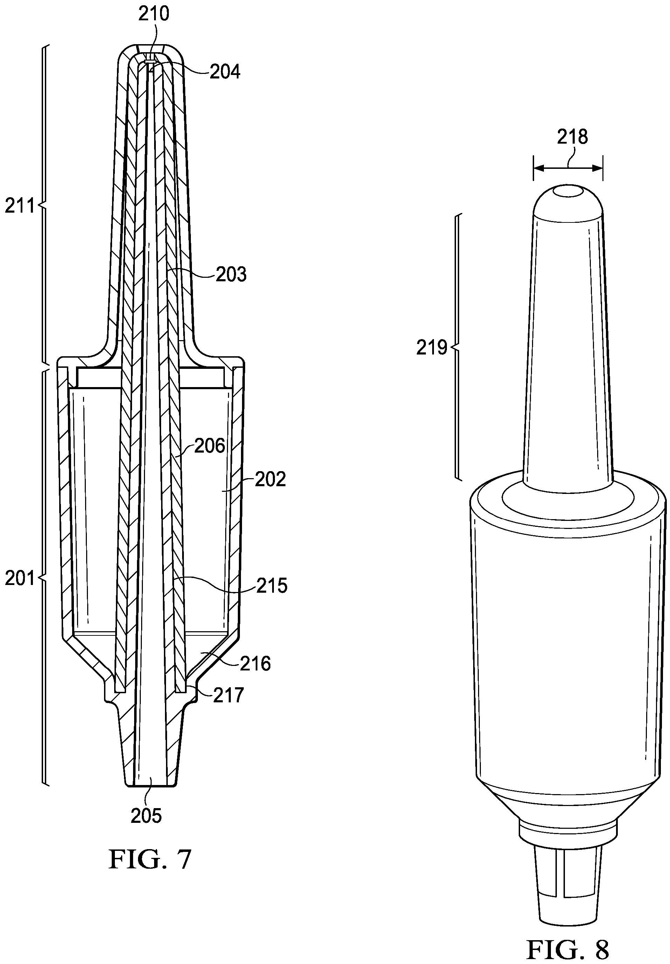

**Figure 7** is a schematic cross section view of the assembled nasal irrigator in accordance with the present invention. This view shows the alignment of the canister **201**, insert **206**, and cover **211** and the resulting fluid space **215.** When fluid is in the reservoir **202** and a pressurized air source is introduced to the system via air inlet **205**, a vacuum is created in the space **215** as air exits through outlets **204** and **210**. Because the aerosol exit hole **210** in the insert **206** is larger than the exit hole **204** of the air exit port **203**, when air is forced through the air exit port **203** at an appropriate volume and speed it creates a venturi effect as the pressurized gas is expelled, thereby drawing fluid in the reservoir **202** up into the space **215** between the insert and air outlet. When the fluid reaches the airstream between the exit holes **204**, **210**, it is atomized in the airstream to create an aerosol. This aerosol is sufficiently dispersed within the nasal cavity above the inferior turbinate so as to the reach the upper nasal cavity.

The aerosol exit **210** in the insert **206** is small enough to ensure that a mist is created yet large enough to ensure that the hole can be chamfered on the outer side to reduce agglomeration of the mist particles upon exit. The aerosol exit hole **210** is chamfered so that the walls of the exit are angled away from a central axis of the hole such that the angle is greater than that of the aerosol plume. This chamfering reduces agglomeration of particles on the walls of the aerosol exit hole **210**, resulting in uniformity of particle size across the resultant aerosol plume.

The base of the insert 206 sits in a groove 217 at the base of the canister 201, ensuring that all fluid is drawn from the bottom of the canister.

The irrigator components of the present invention can be made from materials such as rigid plastic, glass, metal, ceramic, carbon fiber or other rigid material, an elastomer plastic, or some combination thereof.

Figure 8 shows a perspective view of an assembled nasal irrigator in accordance with the present invention. By maintaining a sufficiently narrow nozzle assembly **218**, and a sufficiently long and smooth cover **219**, the device can be easily and atraumatically inserted into the nose of the patient so that the nozzle **218** extends to or above the nasal valve. The device is then angled by the user to obtain the best distribution based on the user's anatomy. The mist enters the nasal cavity independent of the patient's breathing.

The nasal irrigator of the present invention may also include a feature that guides the user to angle the spray into the nose to a set angle of between 0 and 90 degrees from the vertical plane of the face (defined as the front of the face from the chin to the forehead). For example, one embodiment of the nasal irrigator includes a setoff that sets a specific angle of 30 degrees from the vertical plane of the face. In another embodiment, the setoff angle is 60 degrees from vertical, and in another embodiment the setoff angle is 45 degrees from vertical. The setoff described above is removable to accommodate various size faces and noses.

The method of nasal irrigation of the present invention uses a variable particle size up to 100 microns under a pressure of 1-15 psi (0.069 - 1.0345 bar), creating a pressurized airflow that enables the resultant air-mist stream to reach the whole nasal cavity independent of the patient's breathing. The resultant aerosol mist reaches the area of the nasal cavity above the inferior nasal turbinate or chonchae to ensure that the mist reaches the areas of the sinus ostia to clear this area of the nasal cavity and enable the natural mucociliary flow to clear the sinuses.

By adjusting the size of the exit holes **204** and **210**, the air-fluid mixture can be calibrated to achieve nasal irrigation within a short period of time, without the need for the fluid to exit the nostrils at the time of irrigation, and with a particle size that is designed to loosen the mucous or to enter the sinus cavities, as desired by the end user. In many applications, ideally a mist of 20 microns is delivered at a rate of 0.5 ml per second.

The aerosol mist itself is typically medicated with at least one, and often two or more therapeutic agents. Possible therapeutic agents for use in the medicated mist, either alone or in combination include antibiotics, antifungal agents, corticosteroids and mucolytic agents. The mist may also be medicated with a neurologically-active agent targeting the central nervous system through the cranial nerves innervating at least a portion of the nasal cavity as well as systemically-active agents.

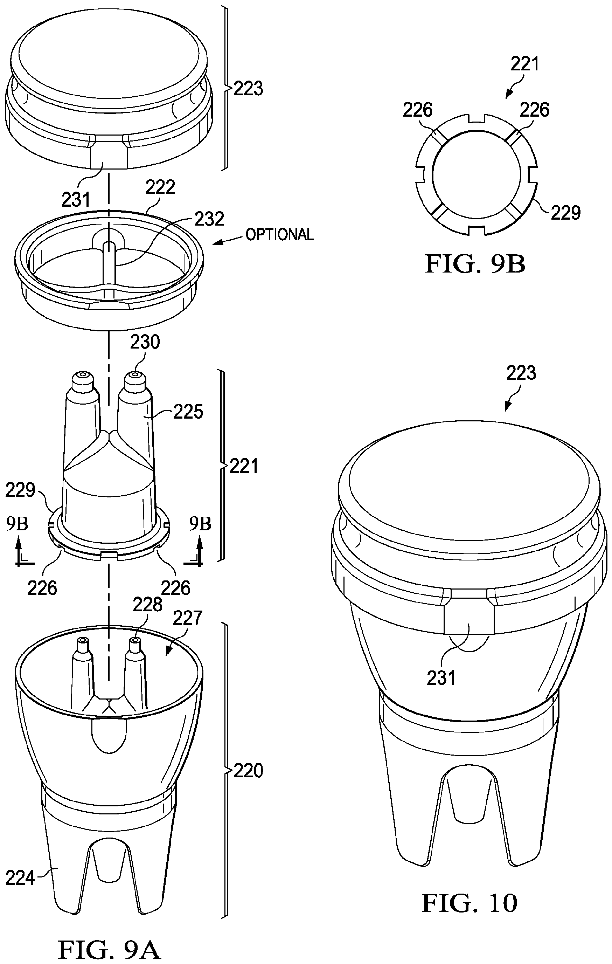

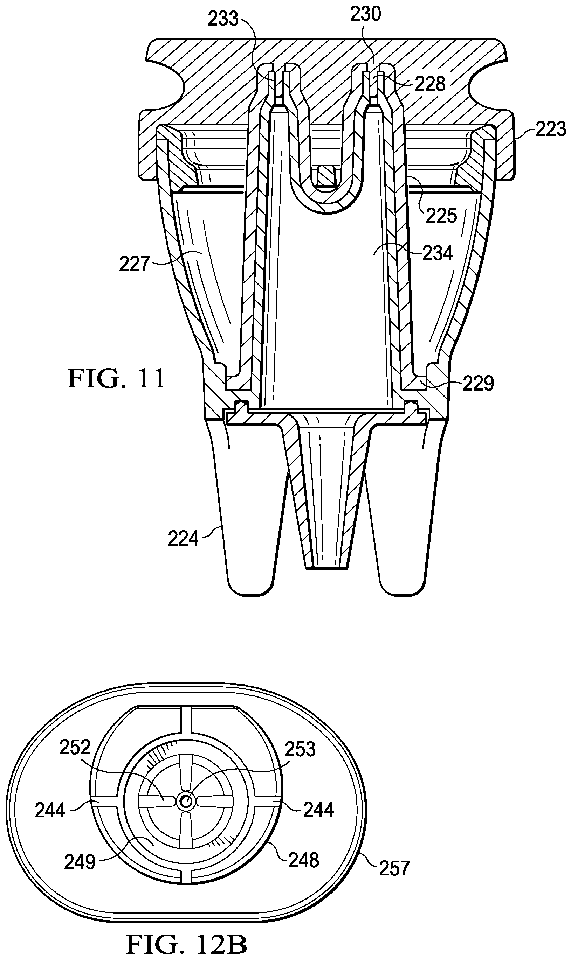

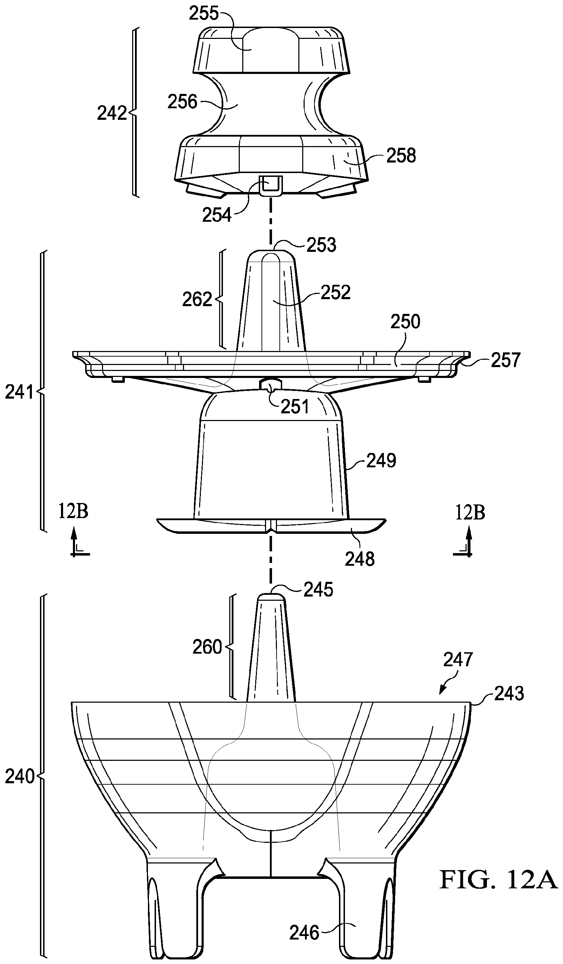

**Figure 9a** is an exploded view of an improved nasal irrigator device according to one embodiment of the present invention. The device comprises a main canister **220**, an insert **221**, and a cap **223**. The main canister **220** and the insert **221** comprise many of the same characteristics of the irrigator described with relation to **Figure 1**. The main canister **220** comprises a rim surrounding a reservoir **227**, which can hold up to 50 mL of fluid. While the reservoir is depicted as substantially circular, it should be appreciated that the reservoir may comprise any shape. In one embodiment, the reservoir comprises an oval shape. As previously described with respect to **Figure 1**, the main canister **220** also comprises an air chamber that terminates into at least one air exit port **228**. In one embodiment, as depicted in **Figures 9-11**, the air chamber of the canister terminates into two air exits ports **228** (one for each nostril). In another embodiment, as best depicted in **Figures 12-14**, the air chamber of the canister terminates into only one single air exit port.

As described above with respect to **Figure 1**, each air exit port **228** has at least one hole of between 0.020" and 0.060" (0.508 mm - 1.524 mm) in diameter and a web-thickness or hole length of between 0.030" and 0.200" (0.762 mm - 5.08 mm). In addition, as with the embodiment of **Figure 1**, on the bottom of the main canister **220** is a foot section **224** that includes at least one foot for stability and an air inlet (as depicted in **Figure 11**) for the admission of pressurized air to create the air stream through air exit ports **228**. The foot section **224** enables the canister **220** to remain standing on its own when set on a substantially horizontal surface and is designed to fit into a standard docking port of an air compressor pump to enable the device to remain upright in a hands-free manner so as to remain filled with the air supply tube attached.

The insert **221** comprises a base **229** that fits within the canister **220** and sits just off the bottom of the reservoir **227**. In one embodiment, as depicted in **Figure 9**, the base **229** is circular. However, the base may comprise any number of shapes so long as it fits within the canister. The insert **221** further comprises a fluid channel **225** that fits over the air exit port **228**, said fluid channel **225** comprising a tube portion ending in a common bell housing **234** above the base. In one embodiment, the insert comprises two fluid channels. In another embodiment, described below, the insert comprises one fluid channel.

As best depicted in **Figure 9b**, the bottom face of the base **229** of the insert **221** comprises at least one groove **226** that forms a communication channel between the canister and the common bell housing of the insert. The groove **226** extends from the outside of the base to the inside of the insert. The base should comprise at least one groove but may also comprise more than one, as depicted in **Figure 9b****.** The number of grooves as well as the width and depth of the groove will help regulate the flow of fluid up to the point that the airflow takes over the upper limit of flow. In one embodiment, the grooves may range in width from about 0.005" to about 0.150" (0.127 mm to about 3.81 mm). In one embodiment, the grooves may range in depth from about 0.001" to about 0.050" (.0254 to about 1.27 mm). The fluid channel **225** is larger in diameter than the air exit port **228,** thereby providing a small space between the outer surface of the air exit port **228** and the inner surface of the fluid channel **225** that allows fluid from said reservoir **227** to be drawn through the communication channel and upward between the air exit port **228** and the fluid channel **225** such that the fluid is expelled as a mist in an aerosol plume through an exit hole **230** in the fluid channel due to a venturi effect created by the introduction of pressurized air from the air exit port.

In one aspect, the canister **220** and the insert **221** are preferably affixed together such that the insert **221** and the canister **220** together form an integral piece. As used herein, "affix" relates to a secure attachment between the canister and insert and may include both permanent bonding and temporary bonding, which may only be subsequently manually separated. Preferably, the affixing of the insert and canister will not interfere with or negatively affect the communication channel(s) formed by the grooves in the bottom face of the insert. In one embodiment, the insert **221** is permanently affixed or bonded to the canister **220** at the bottom face of the insert. The bond may be formed by any means known in the art including without limitation use of a solvent bond, glue UV-cured adhesives, mechanical attachment, heat forming, or radiofrequency or ultrasonic welding. In another embodiment, the canister **220** and the insert **221** may mechanically mate together, such as with a friction fit or a snap fit, to form a temporary connection between them that can be subsequently separated by the user as desired.

In yet another embodiment, where the insert comprises two fluid channels, the nasal irrigator may further comprise a cross bar component **222** having an edge that fits around the rim of the canister. The crossbar component may comprise a single crossbar **232** that extends from one edge of the component **222** to another edge, dividing the component **222** into two substantially equal halves, as depicted in **Figure 9a** for example; or it may comprise a crossbar that extends from one edge to one or more other edges at a different locations around the circumference, dividing the enclosed space into multiple areas. In such embodiments, the crossbar component **222** may be permanently affixed or bonded to the rim of the canister **220**, thereby affixing the insert **221** to the canister **220**. The bond may be formed by any means known in the art including without limitation use of a solvent bond, glue UV-cured adhesives, mechanical attachment, heat forming, or radiofrequency or ultrasonic welding.

Covering the canister **220**, insert **221**, and optional crossbar component **222** is a cap **223** without holes therethrough. As depicted in **Figure 10**, a cap **223** fits over the rim of the canister **220** and covers the tube portion of the insert, plugging the exit hole **230** of the fluid channel **225** and the air exit port **228** to form an airtight, hermetic seal for the irrigator device, preventing the leakage of the fluid from the reservoir. The cap may further comprise an alignment feature or thumb hold **231** along its outer edge, which may align with a similar alignment feature or thumb hold on the exterior of the canister **220**. Thus, the irrigator in one embodiment allows for sterile or non-sterile drug storage and serves as a carrier for the transport or shipment of medication or irrigation fluid.

**Figure 11** is a cross sectional view of an assembled nasal irrigator comprising a canister **220**, insert **221**, optional crossbar component, and cap **223**. As best shown here in **Figure 11**, the cap **223** may comprise sealing plugs **233** recessed within the cap, which extend through both the exit hole **230** of the fluid channel **225** and the air exit port **228**. In one embodiment, the sealing plugs **233** may be comprised of an expandable material, which will expand once removed from the top of the irrigator device. In another embodiment, the cap may be threaded and include a gasket to form a compression seal. When ready for use, a user can remove the cap and connect an air supply to the air inlet beneath the reservoir.

A method of forming a disposable nasal irrigator in comprises the steps of providing a canister **220** with an air exit port **228** and a rim surrounding a reservoir **227** for holding fluid; providing an insert **221** with a base **229** that fits within the canister **220**, the insert **221** comprising a fluid channel **225** that fits over the air exit port **228**, said fluid channel comprising a tube portion ending in a common bell housing **234** above the base, said base comprising at least one groove **226** along its bottom face forming a communication channel between the reservoir **227** of the canister **220** and the common bell housing **234**, wherein the fluid channel **225** is larger in diameter than the air exit port **228**, thereby providing a small space between the outer surface of the air exit port **228** and the inner surface of the fluid channel **225** that allows fluid from said reservoir **227** to be drawn through the communication channel and upward between the air exit port **228** and fluid channel **225**; and affixing the canister **220** together with the insert **221**, thereby forming one integral structure.

The providing steps (a) and (b) can comprise the step of manufacturing the canister or the insert, or both the canister and the insert. The manufacturing can be performed by any means known in the art including without limitation molding, forming, shaping or any combination thereof. The providing step (a) may also comprise the step of obtaining the canister from any manufacturer or vendor, for example. Similarly, the providing step (b) may comprise the step of obtaining the insert from any manufacturer or vendor. By way of example, in one embodiment, the insert may be permanently attached to the canister along its base **229**. Preferably, the bond would be formed such that the groove **226** remains a communication channel. Thus, the bonding should not substantially block or plug the groove **226**. In one embodiment, the insert is bonded or permanently attached along its bottom face to an interior side of the canister. A suitable solvent bond includes, for example, any plastic adhesive including without limitation ABS, acrylic, polystyrene, and polycarbonate solvents such as cyclohexanone. With the insert and canister forming one integral structure, fluid may be inserted into the reservoir **227** and the cap **223** can be placed over the rim of the canister to seal the fluid within the irrigator device for transport or shipment.

**Figure 12a** depicts an exploded view of another embodiment of a nasal irrigator. Similar to the above devices, the nasal irrigator comprises a main canister **240** with an air exit port **245** and a rim **243** surrounding a reservoir **247** for holding fluid. The air exit port **245** extends beyond the rim **243** of the irrigator and has at least one exit hole at the top sufficient to deliver an airstream that is able to atomize fluid and deliver an aerosol. The main canister may also comprise the foot section **246** for stability. In addition, if desired, the canister may comprise one or more horizontal marks or lines to indicate specific fluid levels.

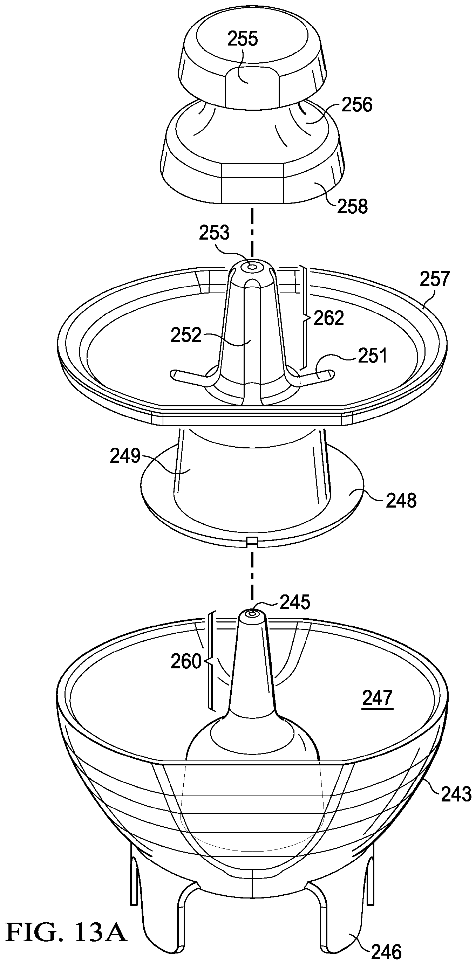

**Figure 12b** depicts an embodiment of the insert **241** with a base **248** that fits within the main canister **240**, wherein the insert has a bottom face with at least one groove **244** to form a communication channel between the canister **240** and the common bell housing **249**. In one embodiment, as depicted in **Figure 12b**, the groove at the bottom of the insert **241** may extend from the outside edge of the bottom face to a peripheral groove surrounding the opening of the common bell housing. The insert further comprises an extension **250**. As depicted in **Figures 12a** **and** **13a**, in one embodiment, the extension **250** protrudes outwardly from the midsection of the insert **241** above the common bell housing **249**. In other embodiments, however, the extension may also extend from another point along the insert, from the common bell housing to any point closer to the exit **253** of the fluid channel. The extension **250** forms a top, or lid, to the canister **240** that mates with the rim **243** of the canister. In one embodiment, the extension comprises a downward concave shape relative to a plane substantially perpendicular to the fluid channel; or, relative to the top surface of the lid. In one embodiment, the extension comprises a two-step diameter **257** to mate with the rim **243**. The insert **241** further comprises one or more apertures **251** around the fluid channel, each of the apertures lining up with a vertical groove **252** along the exterior of the fluid channel **262**. The groove **252** runs vertically from a point below the exit hole of the fluid channel **253** down to an aperture **251** in the extension **250**. During use, the deflected fluid will begin to flow back down the vertical groove **252**. The aperture **251** communicates with the inner chamber formed by the mating of the main canister **240** and insert **241**. As fluid exits the inner reservoir, a vacuum is created that actually pulls the deflected fluid back into the reservoir **247** through the aperture **251**, thereby ensuring maximum usage and minimized waste of the fluid.

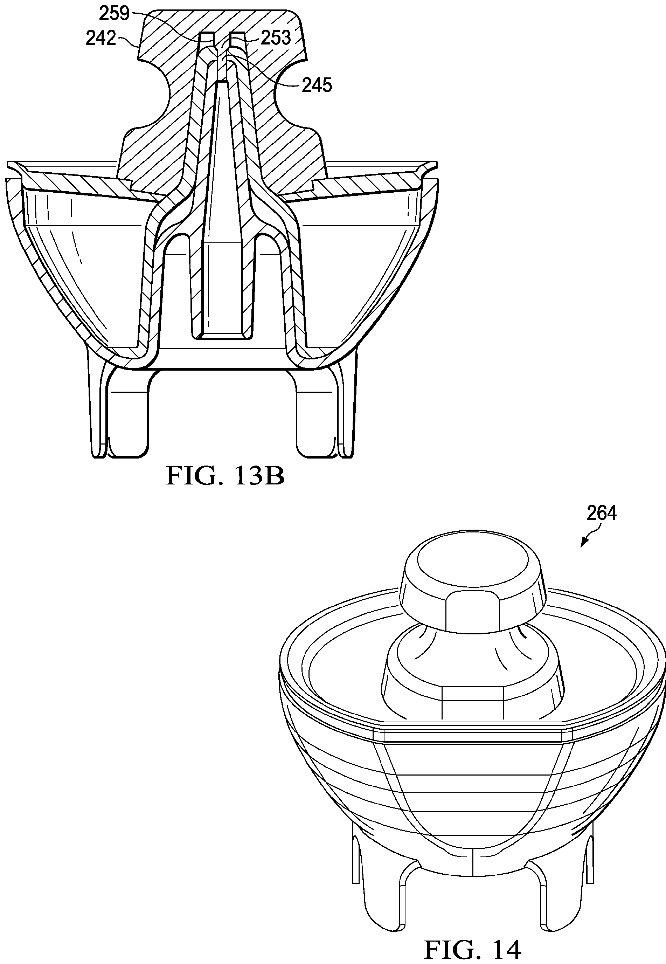

The irrigator further comprises a cap **242** without holes that fits over and inserts into the fluid channel **253** and the air exit **port 245** to seal the reservoir from the air exit and fluid exit. The cap comprises an elongated portion **256** to ensure a good fit over the tube portion. Optionally, the cap may comprise a flattened edge **255** to help with alignment with the apertures **251** of the insert **242** and also help with the grasping the cap **242**. The bottom portion **258** of the cap mates with a portion of the top face of the extension. Thus, as best depicted in **Figure 12a**, the bottom face of the cap **242** is relatively upwardly convex in one embodiment to mate with the downwardly concave extension **250**. The cap **242** further comprises one or more projections **254** on its bottom face, which mates with the apertures **251** of the extension. In particular, the projection **254** aligns with and seals the aperture **251** when the cap **242** is placed over the insert **241**, as best shown in **Figure 14**. Thus, the number of projections **254** on the bottom face of the cap **242** should equal the number of apertures **251** in the insert **250**. As best depicted in **Figure 13b**, the cap further comprises a sealing plug **259** that projects into and fits within the exit hole of the fluid channel **253** in the insert **241** and the air exit port **245,** thereby sealing the nasal irrigator.

Similar to the embodiments described above with regard to **Figures 9-11**, in order to make a disposable device in accordance with one aspect of the present invention, the canister **240** and the insert **241** are affixed together such that the insert **241** and the canister **240** together form an integral or single piece. In embodiments comprising an extension **250** extending from the insert to the rim of the canister (as depicted in **Figures 12-13****)**, the extension may form a top that mates with the rim of the canister and the edges of the extension may be permanently affixed to the rim of the canister. Thus, in one embodiment, it is the extension that is permanently affixed to the rim of the canister by way of bonding, for example. In another embodiment, the extension may form a top that mates together with a portion of the canister. A suitable solvent bond includes, for example, any plastic adhesive including without limitation ABS, acrylic, polyacetal, polyethylene, polyester, polypropylene, polystyrene, or polycarbonate solvent, UV-cured adhesive, heat or ultrasonic welding or over molding of materials. Bonding with such materials can be performed by any means known in the art. Having the insert and canister as a single integral piece, fluid may be inserted into the reservoir **247** and the cap **242** can be placed over the exit hole **253** and the aperture(s) **251** of the insert **241** to seal the fluid within the irrigator device for transport or shipment. The cap sits over the tube portion of the fluid channel and the fluid within the reservoir remains sealed within the irrigator device until ready for use. **Figure 14** depicts an assembled, sealed device **260** ready for transport.

As with the above embodiments, the orifices of the fluid channels should be positioned relative to the air exits so as to create a venturi effect with the pressurized gas expelled from the gas tubes. Thus, the affixing step should account for this positioning. Because the fluid channel exits in the insert are larger than the air exits, when air is forced through the air exits at an appropriate volume and speed, fluid in the reservoir is drawn up into the space between the insert and air exits ports. When this fluid meets the subsequent airstream it is atomized into particles conducive to deposition in the upper airway.

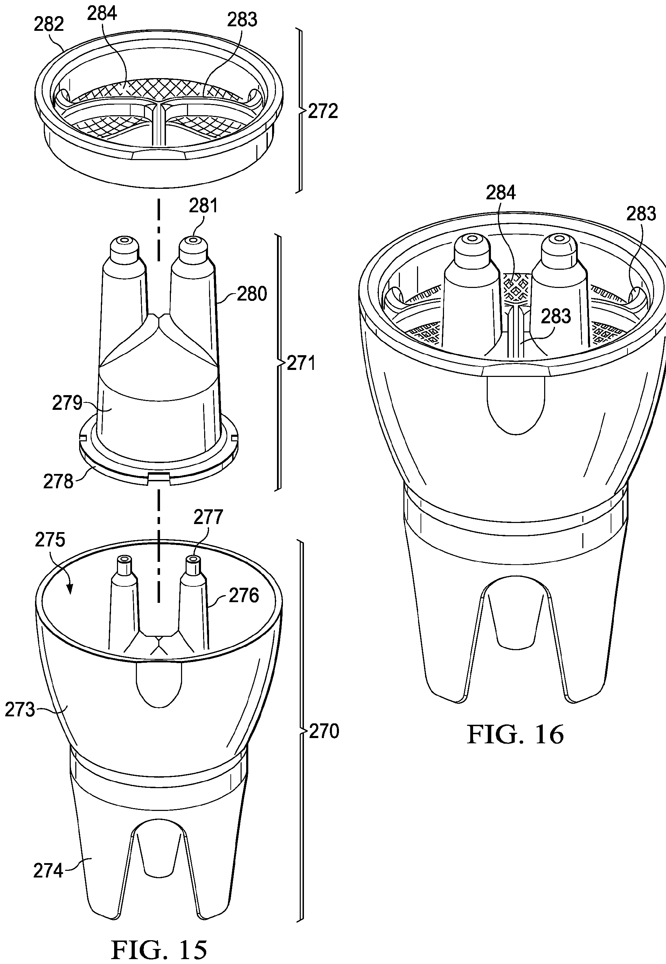

**Figure 15** is an exploded view of an embodiment of a nasal irrigator device comprising a canister section **270**, an insert **271**, and a filter **272**. Similar to the above devices, the canister section **270** comprises a canister **273** with reservoir **275** and an air exit port **276** having an exit hole **277**. The canister section **270** also comprises one or more feet **274** beneath the canister **273**; and the insert **271** comprises a base **278** that fits within the reservoir of the canister and at least one fluid channel **280** with an exit hole **281**. As described above, the insert and canister section once formed, shaped, molded or obtained, are affixed to one another.

In one embodiment, the nasal irrigator device further comprises a filter component **272** that may be inserted over the insert **271**. The filter component **272** comprises a filter **284** comprised of a mesh structure with holes small enough to prevent any particulate matter or mucus that runs out of the nose from entering the reservoir **275**, while allowing the irrigating or medicating fluid to run back into the reservoir **275** to be re-circulated or re-used. Suitable materials from which to create the filter are plastic, metal, carbon fiber, or other fiber. In embodiments comprising more than one fluid channel, the filter component also comprises a crossbar component **283**. In one embodiment, the crossbar **283** is an integral part of the filter component **272**. However, it should be understood that the crossbar **283** could also form a separate component, which is detached from the filter, and remains optional.

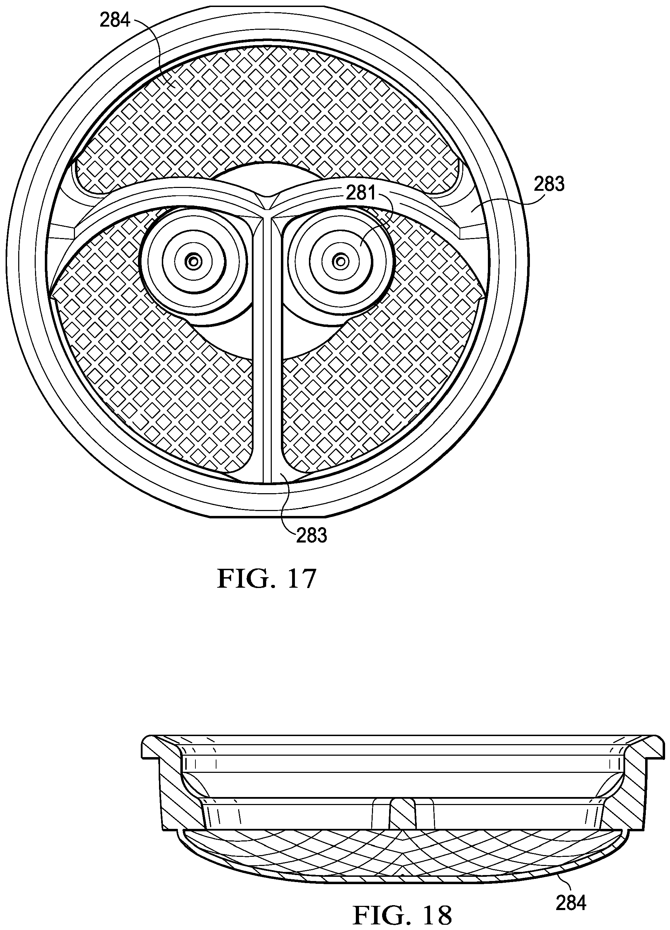

**Figure 16** is a perspective view of an assembled irrigator having an insert having two fluid channels **280** and a filter **284** with the optional crossbar **283**, wherein the insert is affixed to the canister to form one single integral structure. As described above, in one embodiment, the insert is affixed to the canister by way of bonding. The bonding may comprise the joining of the bottom face of the insert base to the canister or the joining of the periphery of the base to the canister. In one embodiment, the insert may be affixed to the canister by permanently bonding the periphery **282** of the filter to the rim of the insert. As best depicted in **Figure 17**, the filter **284** surrounds the tube portion **280** of the insert and extends from the rim of the canister to the tube portion **280**, substantially covering the opening of the canister such that when in use, the filter prevents particulate matter from entering the reservoir.

With reference to **Figures 12** **and** **13**, where the nasal irrigator comprises an extension, in one embodiment, a filter entirely covers or fits within the apertures **251** in the extension **250** to similarly keep particular matter out of the reservoir and separate from the fluid for re-circulation. The filter may slide over the fluid channel of **241** or may be bonded over or under the apertures **251** or even molded into the insert **241.**

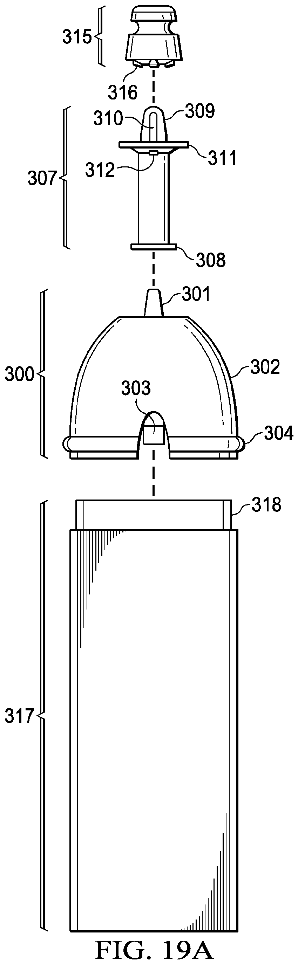

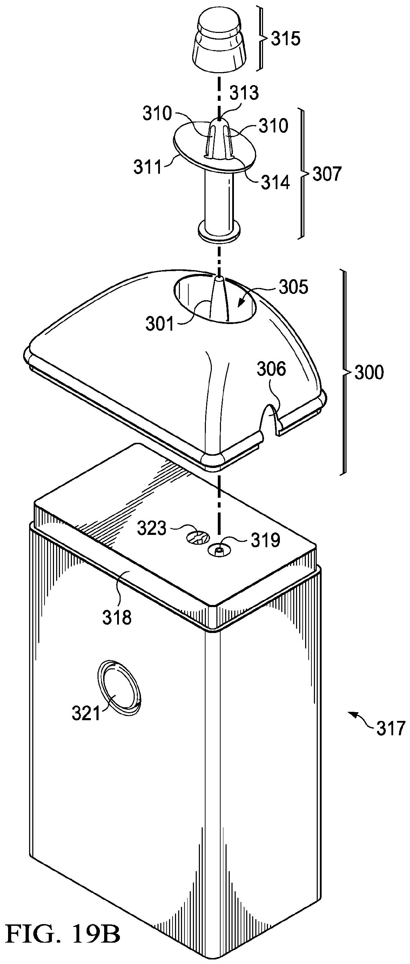

**Figures 19A** and **19** **B** show an exploded view of a portable nasal irrigator in accordance with an embodiment of the present invention. The portable irrigator comprises four sections. The first major section is the main canister **300**, which comprises a reservoir **305** for receiving fluid. The main canister **300** further comprises an air exit port **301**. As depicted in the figures, the air exit port **301** may extend above the top edge of the main canister **301**. However, in alternate embodiments (not shown), the air exit port **301** may be even with or recessed within the edge or portions of the edge of the main canister **300**. While the reservoir is depicted as substantially circular, it should be appreciated that the reservoir may comprise any shape. In one embodiment, the reservoir comprises an oval shape. Preferably, the reservoir should be shaped to allow for the receipt of a maximum amount of fluid.

Returning to the embodiment depicted beginning at **Figure 19A**, the main canister further comprises a curved wall **302** surrounding the opening to the reservoir **305**. The curved wall **302** comprises a convex shape that extends downwardly around the periphery of the opening into a bottom generally rectangular opening configured to mate with a pressurized air supply, as further discussed below. When viewed from below, the main canister **300** thus comprises a generally hollow portion surrounding the reservoir portion **305**.

The second major section of the portable irrigator is the insert **307**, which comprises a base **308** that fits within the reservoir section **305** of the canister. As depicted in **Figures 19A** and **19B**, the base **308** is circular. However, the base may comprise any number of shapes so long as it fits within the canister. The insert comprises a fluid channel **309** with one end at the bottom of the reservoir **305** and one end that is positioned in the airstream so that the airstream creates a negative pressure in each tube that draws fluid into the airstream where it is atomized. The end positioned in the airstream comprises an exit hole **313**. The fluid channel **309** is slightly larger in diameter than the air exit port **301** of the main canister **300**, thereby providing a small space (preferably 0.0001" to 0.010" (0.00254 - 0.254 mm)) between the outer surface of the air exit ports and the inner surface of the fluid channels. This space allows fluid from the reservoir **305** to proceed upward between the air exit port **301** and the fluid channel **301** until being expelled by pressurized air. When the insert **307** is installed in the main canister **300**, the orifice **313** of the fluid channel **301** is positioned relative to the air exit **301** so as to create a venturi effect with the pressurized gas. Because the fluid exit in the insert **313** is larger than the air exits **301**, when air is forced through the air exits at an appropriate volume and speed, fluid in the reservoir **305** is drawn up into the space between the insert and air exit port. Thus, when this fluid meets the subsequent airstream it is atomized into particles conducive to deposition in the upper airway. The airstream is sufficient to penetrate the nasal cavity above the inferior turbinate so as to deposit the fluid and provide a washing, irrigation, or deposition to the upper reaches the nasal cavity. The fluid channel size may be adjusted to change the particle size of the mist.

The insert **307** may be keyed in at least one location with the reservoir **305** to ensure that the insert does not rotate in relation to the exit port **301** of the main canister **300** and to aid in centering of the insert **307** and its fluid channel **309** on the air exit port **301**. In one embodiment, the insert may also include a feature to ensure that it is inserted into the main canister in only one orientation.

At least one channel is located in the bottom of the insert **307** to act as a conduit for fluid from the reservoir **305** to enter the base **308** of the insert. As best depicted above in **Figures 9b** **and** **12b**, the bottom face of the base **308** of the insert **307** comprises at least one channel or groove that forms a communication channel between the canister and the insert. The groove extends from the outside of the base to the inside of the insert. The base should comprise at least one groove but may also comprise more than one, as depicted in **Figure 9b**. The number of grooves as well as the width and depth of the groove will help regulate the flow of fluid up to the point that the airflow takes over the upper limit of flow. In one embodiment, the grooves may range in width from about 0.005" to about 0.150" (0.127 mm to about 3.81 mm). In one embodiment, the grooves may range in depth from about 0.001" to about 0.050" (0.0254 to about 1.27 mm).

The canister **300** and the insert **307** may or may not be affixed together to form one integral piece. The bond may be formed by any means known in the art including without limitation use of a solvent bond, glue UV-cured adhesives, mechanical attachment, heat forming, or radiofrequency or ultrasonic welding. Alternatively, the canister and insert may be affixed together via a mechanical interlocking element such as a friction fit or a snap fit to form a temporary connection.

The insert further comprises an extension **311**. As depicted in **Figures 19A** **and** **19B**, the extension **311** protrudes outwardly from the insert **307.** The extension **311** may extend from any point along the insert to form a top, or lid, to the canister **300**. In one embodiment, the extension substantially covers the opening of the reservoir **305**. In another embodiment, the extension entirely covers the opening of the reservoir **305**. In one embodiment, the extension comprises a downward concave shape relative to a plane substantially perpendicular to the fluid channel; or, relative to the top surface of the lid. In one embodiment, the extension comprises a two-step diameter (not shown) to mate with a rim of the opening. The insert **307** further comprises one or more apertures **314** around the fluid channel, each of the apertures lining up with a vertical groove **310** along the exterior of the fluid channel **309**. The groove **310** runs vertically from a point below the exit hole of the fluid channel **309** down to an aperture **314** in the extension **311**. During use, the deflected fluid will begin to flow back down the vertical groove **310**. The aperture **314** communicates with the inner chamber formed between the main canister **300** and insert **307**. As fluid exits the inner reservoir, a vacuum is created that actually pulls the deflected fluid back into the reservoir **305** through the aperture **314**, thereby ensuring maximum usage and minimized waste of the fluid.

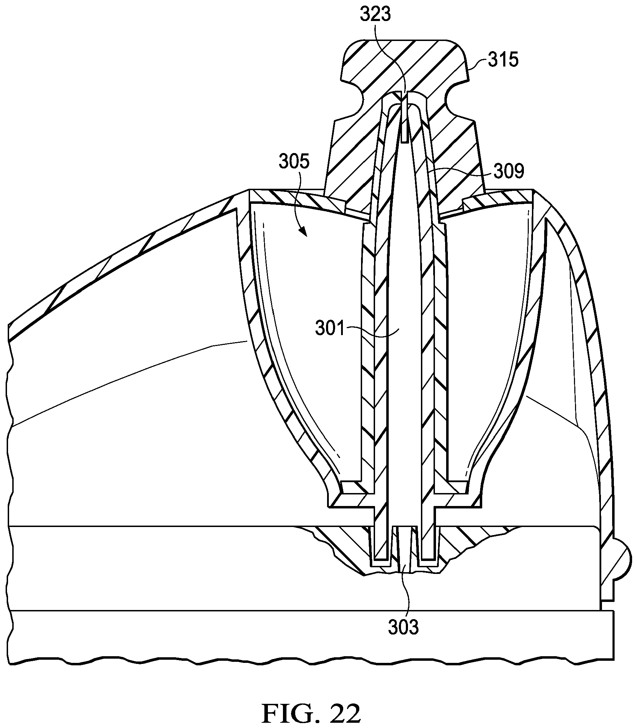

Another section of the portable nasal irrigator is a removable cap **315** of the nasal irrigator. The cap **315** comprises no holes and fits over and substantially covers the fluid channel **309**. Optionally, the cap may comprise a flattened edge (as shown above in **Figure 12A****)** to help with alignment with the apertures **314** of the insert **307** and also help with the grasping the cap **315**. The bottom portion of the cap should mate with a portion of the top face of the extension. The cap **315** further comprises one or more projections **312** on its bottom face, which mates with the apertures **314** of the extension. The number of projections **312** on the bottom face of the cap **315** should equal the number of apertures **314** in the insert **307**. As best depicted in **Figure 22**, the cap comprises a projection or sealing plug **323** that projects into and fits within the exit hole **313** of the fluid channel and extending into the air exit port **301** of the canister to seal the reservoir from the air exit port and fluid channel exit when the cap is placed over the insert.

A fourth section of the portable nasal irrigator is a handheld pressurized air supply source **317** onto which the main canister **300** fits. Preferably, the pressurized air supply source is a handheld air compressor. As shown in **Figure 19B**, the air supply source comprises an air outlet **319**, which connects with the air inlet **303** of the main canister. In one embodiment, the canister snap fits onto the pressurized air supply source **317** to form an airtight seal between the air inlet **303** and the air outlet **319**. In one embodiment, the airtight seal may comprise an O-ring or soft plastic portion between the air inlet **303** and the air outlet **319** (not shown). An air input **320** supplies air to the pressurized air supply source **317** and may comprise a filter to keep out foreign materials. In order to accommodate for the air input **320**, the main canister **300** comprises an air vent **306**, which allows air into the air input **320** without interrupting the airtight seal between the canister **300** and air supply source **317**. The bottom rim **304** surrounding the generally rectangular bottom of the main canister **300** is fashioned to fit onto the pressured air supply source **317** such that no wiring or connecting tubing is required. Thus, unlike previous embodiments, a foot section at the bottom of the main canister is not necessary in order to stabilize the canister on a substantially flat surface. Instead, the pressurized air supply connects directly and immediately with the main canister.

While the pressurized air supply source **317** is depicted as having a generally rectangular shape, the source **317** may comprise any shape so long as it remains portable and capable of directly attaching to the main canister without the use of tubing. In one embodiment, the pressurized air supply source **317** is substantially rectangular. Preferably, the pressurized air supply source comprises an ergonomic shape to increase user comfort. For example, the air supply source **317** may comprise a grasping or gripping portion having a shape that corresponds to a palm of a hand of the user. The gripping portion may be on one side of the air supply source, with a second opposing side substantially flat; or it may comprise curves substantially around the entire periphery of the air supply source such that user may hold the portable device lengthwise with his or her hand around substantially the entire pressurized air supply source **317**. In one embodiment, the air supply source **317** comprises an ergonomic grasping portion. In another embodiment, the pressurized air supply source **317** is substantially rectangular with curves and features that make it easy to hold in the hand. In order to allow for portability of the irrigator device, the pressurized air supply should generally be small enough to easily carry or transport. In one embodiment, the pressurized air supply source comprises a ratio of width: length: depth of about 2.5:3:1. In another embodiment, the pressurized air supply source comprises a ratio of width: length: depth of about 9:15:5. In one embodiment, the pressurized air supply source comprises a ratio of width: length: depth of between about 2.5:3:1 to about 9:15:5. By way of example, in one embodiment, the length may be about 15.5 cm, the width may be about 9.2 cm, and the depth may be about 5.7 cm. It should be recognized that any number of sizes and dimensions is possible while maintaining portability.

The pressurized air supply source **317** may employ an AC/DC power supply. The source **317** is DC-operated and may include a rechargeable internal battery or an external, detachable battery for easy exchange of depleted batteries. The source **317** may further be operated using a power switch **321** capable of turning on the air supply. The switch **321** may be an intermittent switch conveniently located on the air supply source **317** such that a user may conveniently reach it with one of his or her fingers. In one embodiment, the air supply source **317** may also comprise an indicator for the level of charge on the battery (not depicted) or a timer that beeps at timed intervals to deliver medication evenly between nostrils (not depicted). As described above, the pressurized air has a pressure of 0.069 - 1.035 bar and an airflow rate of 1 - 12 liters per minute, producing a fluid delivery rate of 1 - 20 ml per minute.

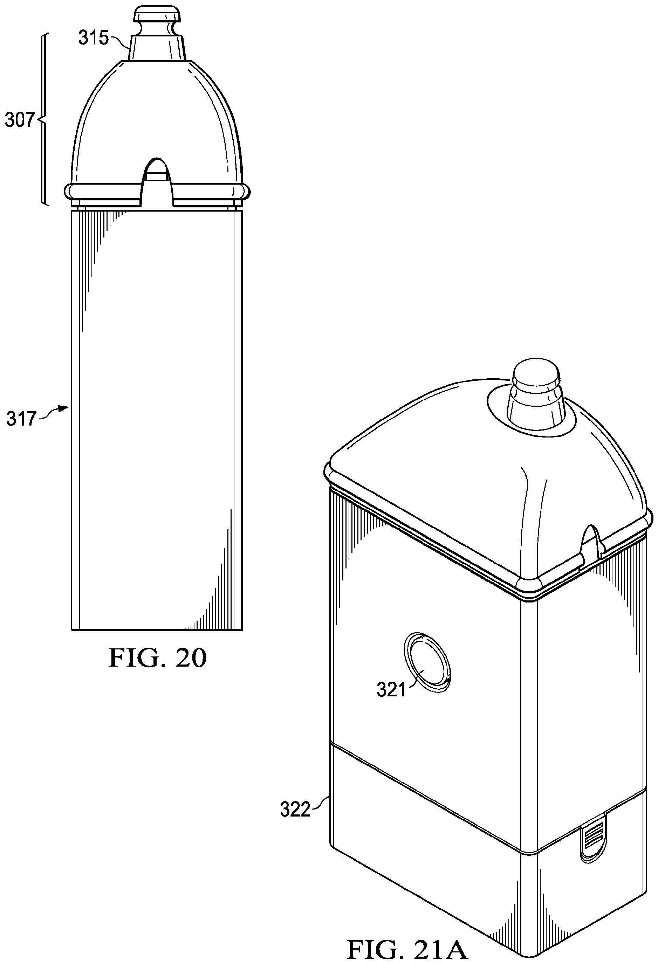

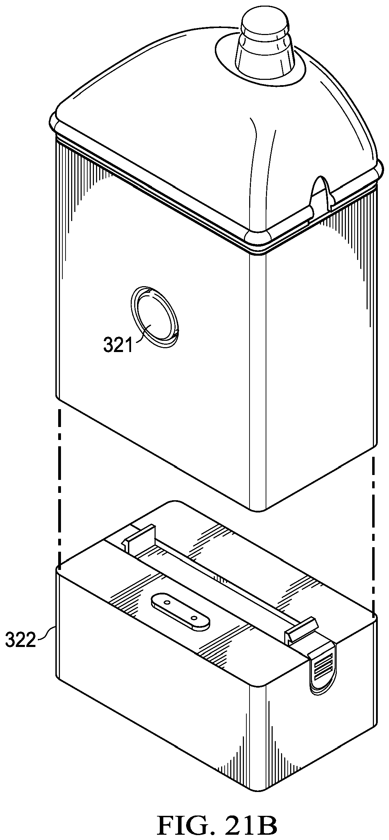

**Figure 20** shows a front perspective view of an assembled portable irrigator as shown in **Figures 19A** and **19B**, with the removable cap positioned over the device. Thus, when fully assembled with the cap in place, the portable irrigator device is completely self-contained, prohibiting any leakage of fluids. As depicted in **Figures 19A** and **19B**, in one embodiment, the pressurized air supply source **317** comprises an internal battery, which may or may not be rechargeable. **Figure 21A** shows a perspective view of an assembled portable irrigator in another embodiment, with a detachable battery compartment **322** for one or more batteries which may or may not be rechargeable. In this embodiment, the battery compartment may detach from a portion of the pressurized air device by way of a switch element. **Figure 21B** shows a perspective view of a portable irrigator as depicted in **Figure 21A**, with the battery compartment **322** detached from the air supply source **317**.

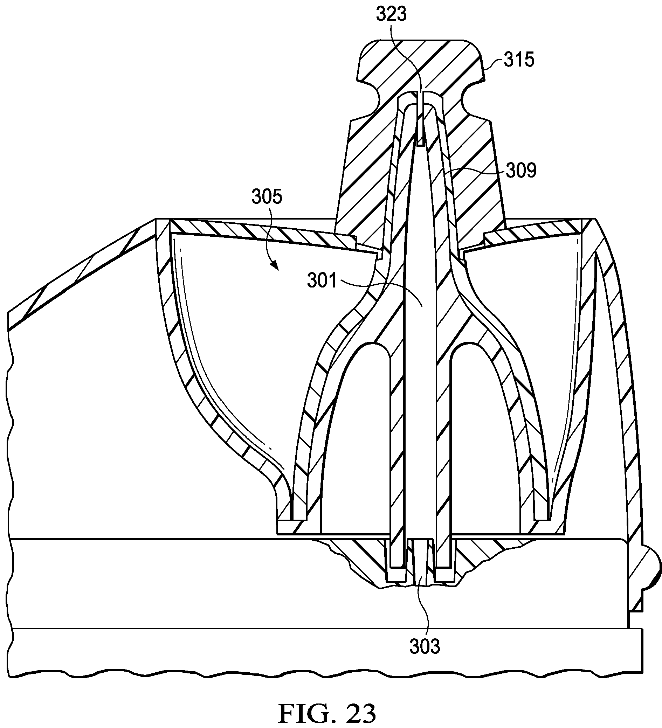

**Figure 22** shows a cross sectional detailed view of the main canister **300**, insert **307** and cap **315** portions in an assembled portable irrigator according to one embodiment of the present invention. As best depicted here, the air exit port **301** and fluid channel **309** form two overlapping, concentric, tapered tubed having the requisite gap or space, as described above, between them in order to allow for the venturi effect. When connected to the pressurized air supply source **317**, the air inlet of the main canister plugs directly the supply source or air compressor by way of its air outlet. An alternate embodiment depicted in **Figure 23** shows that the air exit port **301** and the fluid channel **309** may also include a common bell housing as with previous embodiments.

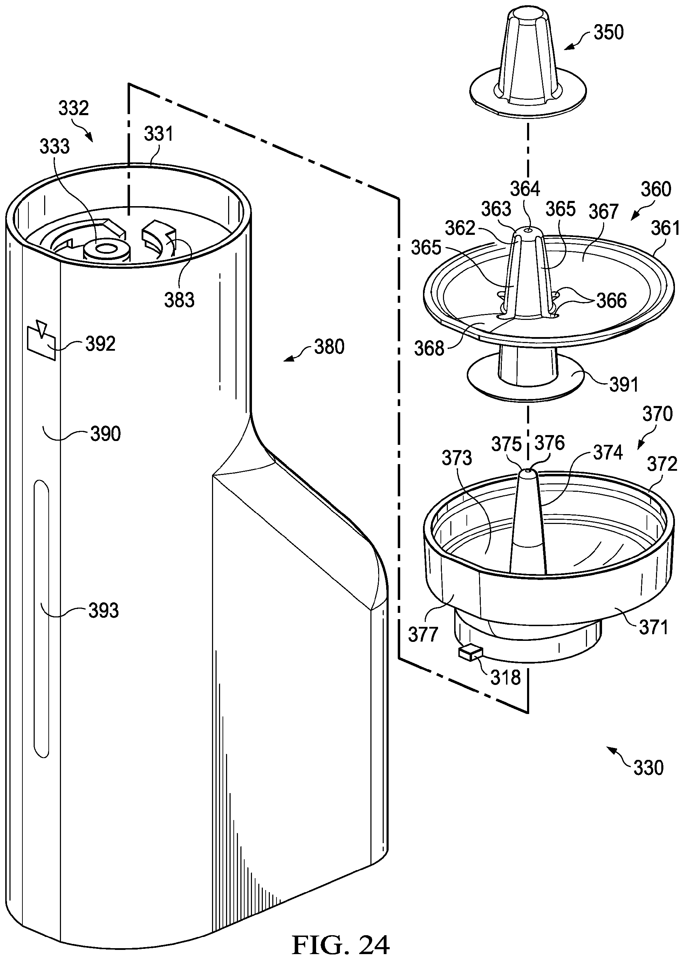

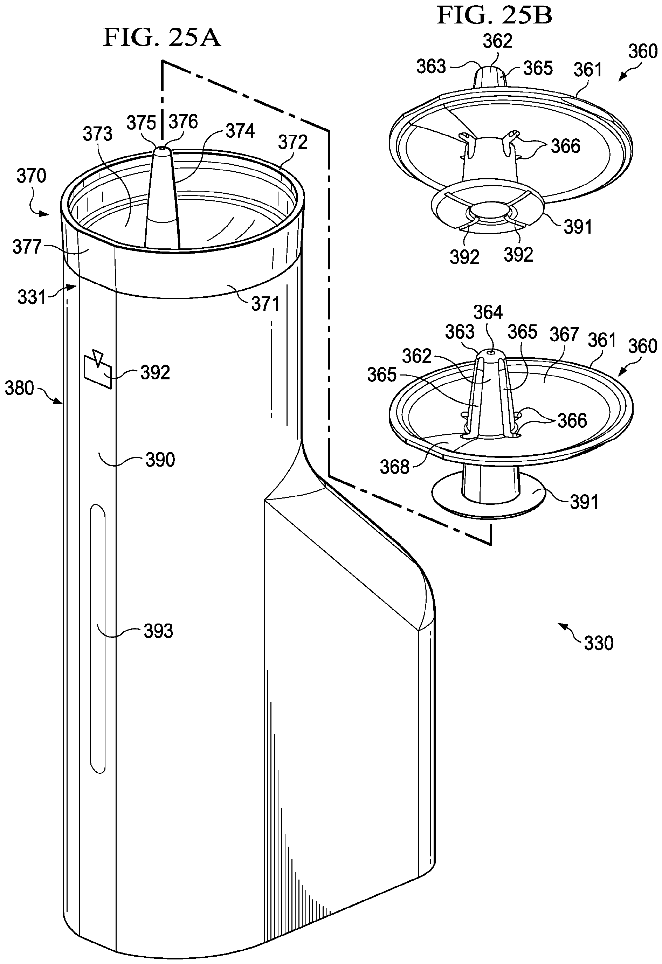

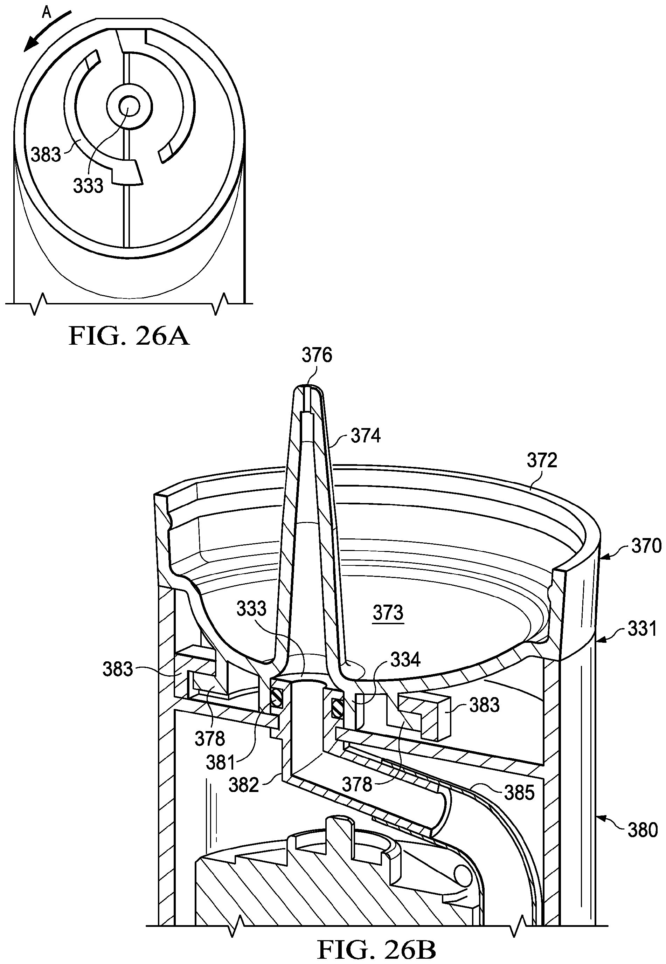

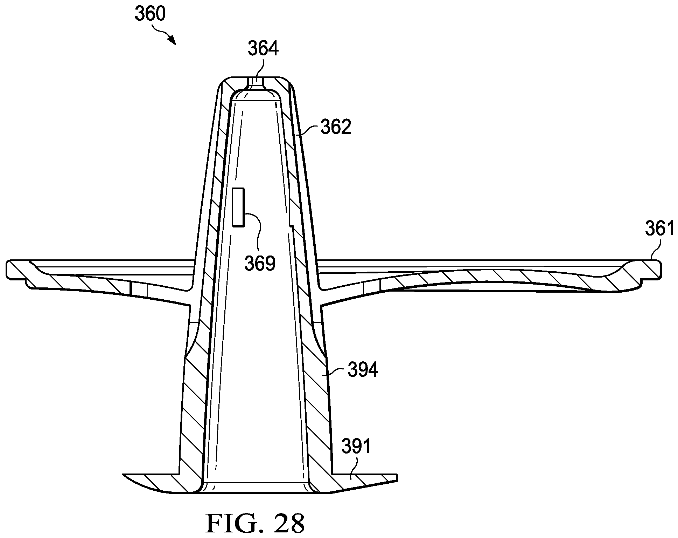

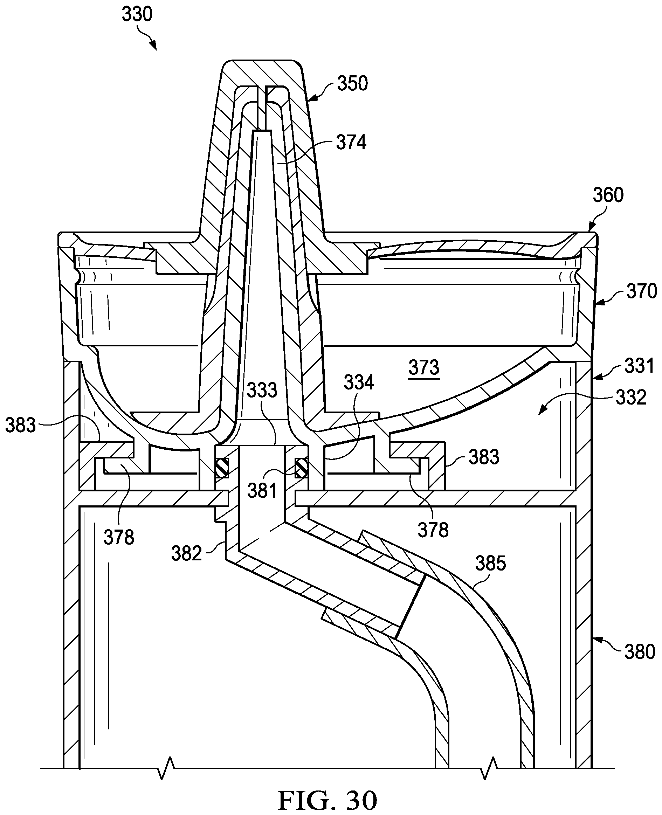

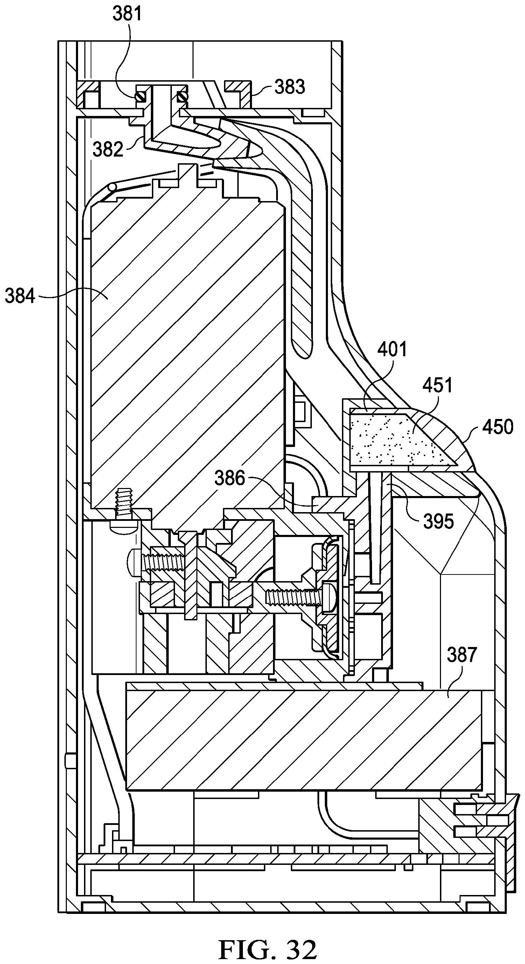

**Figures 24-32** depict another embodiment of a portable irrigator **330**. The portable nasal irrigator **330** comprises: a pressurized air supply source **380** comprising a rim **331** surrounding an opening **332** on one end with an air outlet **333** therein; a canister **370** with a reservoir **373** for holding fluid recessed within the opening **332**, the reservoir **373** surrounding a tube **374** tapering to an air exit port **376**; and an insert **360** comprising a base **391** that fits within the reservoir, an extension **367** above the base **391** protruding outwardly to the canister **370**, and a fluid channel **394** (shown best in **Figure 25B** **and** **28**) that fits over the elongated tube **374**, said fluid channel **394** having a discharge port **364** concentrically aligned with the air exit port **376**, said discharge port **364** located at an uppermost end of the irrigator **330** above the extension **367**, thereby providing a small space between the outer surface of the air exit port and the inner surface of the insert, creating a fluid conduit that allows fluid from said reservoir to be drawn upward between the air exit port and the fluid channel and expelled as a mist in an aerosol plume through exit holes in the fluid channel due to a venturi effect created by pressurized air from the air exit port. The fluid is thus expelled from the reservoir **373** as a mist through the discharge port **364.** The canister **370** comprises: a lip **372** extending above and resting on the rim **331** of the pressurized air supply source **380**; and an air inlet **334** below the reservoir **373**, the air inlet **334** connected to the air outlet **333**; and wherein the pressurized air supply source **380** houses an airflow regulating system. The canister **370** is retained to and sealed together with the pressurized air supply source **380** by the connection of a mating portion **383** and a tab **378**, as further described below. A seal between the air outlet **333** and the tube **374** of the main canister **370** also helps maintain the connection between the canister **370** and the pressurized air supply source **380**, as shown in **Figures 26B** **and** **30**.

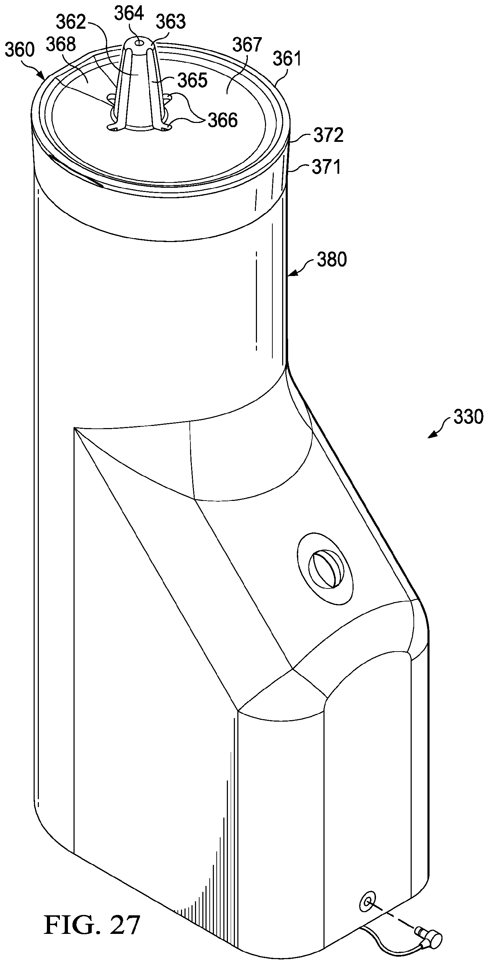

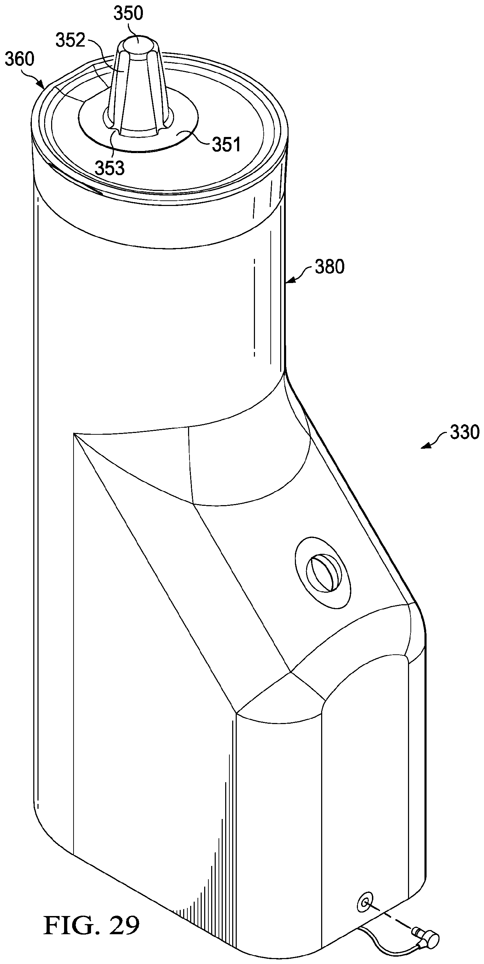

Similar to the above embodiments, the portable irrigator **330** comprises a cap **350**, insert **360**, and a cup or canister **370**. **Figure 24** shows an expanded view of each of these components and their general placement over one another and the pressurized air supply source **380**. **Figure 25** shows the canister **370** connected to the pressurized air supply source **380**, while Figure **27** shows an assembled irrigator **330** with placement of the insert **360** over the canister **370** while connected to the pressurized air supply source **380** for usage by a consumer, and **Figure 29** shows placement of the cap **350** over the insert for storage or transport by a consumer.

As depicted in **Figures 24-25**, the pressurized air supply source **380** is a generally elongated structure comprising a concave opening **332** with an airflow regulating system therein, which will be further described below. In one embodiment, the pressurized air supply source **380** comprises a first larger bottom depth and a second smaller top depth, with an angled surface there between. The shape of the pressurized air supply source thus provides for a grip zone along the length of the pressurized air supply source for consumer handling. The size may be any size that allows for handheld usage. The narrow neck top accommodates single-handed use, while the wider bottom section accommodates a two-handed grip, if desired.

A single membrane **390** along one external side of the pressurized air supply source **380** incorporates a single ribbon connector (not shown) and comprises an indicator light **393**, and a raised dome switch **392** integrated therein, to turn the irrigator on or off. In one embodiment, the switch **392** is an intermittent operating switch. In another embodiment, the switch is a latching switch. In one embodiment (not shown) the switch is discrete from the other components.

In the embodiment depicted in **Figure 24**, the indicator light **393** comprises a light pipe, which may house one or more light therein. However, in other embodiments, the indicator light may comprise any number of shapes or symbols including, for example, any number of icons or shapes to depict battery power. By way of example, the indicator light **393** may comprise a light pipe, a battery icon, or gas gauge, which may comprise a number of rectangles in a line on the switch. An indicator light may indicate when the irrigation device is charging in one embodiment; or, when the irrigator requires charging in another embodiment; or when the device is operating, in another embodiment. The indicator light may illuminate for a sufficient amount of time for a user to recognize a need for charging after use. For example, the indicator light may illuminate for 5-30 seconds after an operation to indicate when charging is required. In one embodiment, the indicator light may illuminate to indicate when charging is needed while there is still sufficient power to run the device for a full expected dose; ensuring the user is aware of the need to charge and avoid a missed dose. In one embodiment, the indicator light is an LED light source. The LED light source may be a single color LED, multicolor LED or multiple LEDs of a single color or of different colors. In one embodiment, the indicator light uses a light pipe **393** to transmit light to the outside of the device.

In one embodiment, the single membrane **390** contains all electrical components externalized to the user except a power jack, which may be optionally used, for example, to power the irrigator or charge a rechargeable battery within the pressurized air supply source or to operate the device when the battery is discharged such that the device cannot be operated with the battery alone. In one embodiment, the irrigator comprises a tethered cover for the power jack designed to reduce fluid and dust ingress to the device when the power supply is not plugged into the device. Optionally, in one embodiment, an audible indicator is incorporated into the pressurized air supply source of the irrigator to indicate a set time of operation, a need for charging, or the initiation of a charge. On an external side, opposite to the membrane **390**, the pressurized air supply source **380** comprises the angled surface having a cover or cap **450** for a filter for incoming air, further described below.

With reference to **Figure 25**, the canister **370** is recessed within the pressurized air supply source **380,** which has a concave opening. More specifically, a bottom portion of the canister rests within the pressurized air supply source once the canister **370** is attached to the pressurized air supply source **380**. A lip **372** extends above the pressurized air supply source **380** and rests on the rim **331** of the pressurized air supply source. In one embodiment, the lip is of an elliptical shape. The lip **372** surrounds the entirety of the top perimeter edge of the canister **370** and prevents spills from the inner volume or reservoir **373**. In one embodiment, the periphery **371** is elliptically shaped to match an elliptical opening of the pressurized air supply source **380**. A flat portion **377** assists with proper placement and alignment of the canister **370** in one embodiment. Thus, the pressurized air supply source **380** and the canister **370** may employ visual or tactile alignments marks to ensure the user is able to easily join the two components. As with the above-discussed irrigator embodiments, within the reservoir **373** is a tube portion **374** that tapers into an air exit port **376,** which is above a rim of the irrigator. In one embodiment, the air exit port **376** comprises a size of between 0.020" and 0.060" (0.508 mm - 1.524 mm) in diameter and a web-thickness or hole length of between 0.030" and 0.200" (0.762 mm - 5.08 mm). In one embodiment, the canister **370** is reusable. In one embodiment, the canister **370** is disposable.

In one embodiment, as best shown in **Figures 24** **and** **26A-B****,** the canister **370** is positively held to the pressurized air supply source **380** by a locking mechanism, which is comprised of at least one tab **378** on the canister **370** that interfaces with a mating portion **383** that captures the tab **378**. The mating portion **383** protrudes from and is located within the concave opening **332** of the pressurized air supply source **380** and may comprise any shape capable of locking with a corresponding tab **378** of the main canister **370**. In one embodiment, the mating portion **383** comprises one or more rounded or curved protruded edges with a thicker end piece at one end under which one or more tabs **378** may lock. One or more tabs **378** on the base of the canister **370**, below the reservoir **373**, then securely fits within the upper end of the pressurized air supply source **380** by way of the locking mechanism. The tab **378** or bottom of the main canister **370** fits within an opening **332** of the pressurized air supply source **380**, and the canister **370** is then turned in the direction of arrow A. The tab **378** then slides under the mating portion **383** until the canister **370** locks into place. The locking mechanism in effect presses the canister **370** into a seal that seals the air channel between the canister **370** and the pressurized air supply source **380**. In one embodiment, the tab 378 has a protrusion, bump or other feature that mates with a similar feature on the mating portion 383 to securely lock the two pieces together during operation and provide a tactile and auditory indication that the mating process is secure and the canister **370** and the pressurized air supply source **380** are properly connected.

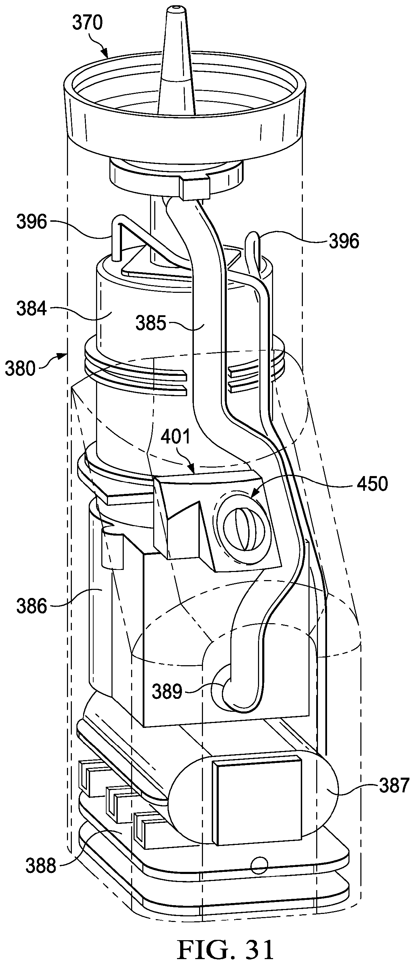

Similar to the portable irrigator described above in **Figures 19-23**, the canister **370** comprises an air inlet **334** at its bottom end below the reservoir **373**, which connects to an air outlet **333** of the pressurized air supply source **380**. In one embodiment, the air inlet **334** comprises an extended bottom portion to aid in sealing with the pressurized air supply source **380**. In one embodiment, air outlet **333** of the pressurized air supply source **380** comprises an air outlet elbow **382** connected to the air inlet **334** of the main canister **370.** An outlet tubing **385** on an opposing end of the elbow **382** connects to a pump **386** as further described below in one embodiment. More specifically, the outlet tubing **385** connects the air outlet **333** to a pump outlet **389**, as shown in **Figure 31**. In one embodiment, the air outlet elbow comprises an angle of between about 30 degrees to about 90 degrees in between its substantially vertical portion extending down from the air inlet **334** and its somewhat horizontal portion connected to the tubing **385**, sufficient to circumvent a motor **384** within the pressurized air supply source **380,** as best shown in **Figure 31**. In one embodiment, an o-ring **381** helps form a seal between the canister **370** and the air outlet elbow **382** of the air outlet **333**.

Referring now to **Figures 27-28****,** when assembled, the irrigator comprises an insert **360** placed over the canister **370**. As shown in **Figure 27**, only the periphery **371** with the rim **372** of the canister **370** is visible when the insert is present for usage of the irrigator. Generally, the insert **360**, also shown in **Figures 28** and **30**, is substantially similar to the inserts described in above embodiments and can thus comprise one or more of the limitations described above. The insert **360** comprises a base **391** that fits within the reservoir **373**, an extension **367** above the base **391** protruding outwardly to the lip **372** of the canister **370,** and a fluid channel **394** that fits over the tube **374**, the elongated fluid channel **394** having a somewhat larger bottom diameter converging up to a smaller diameter at its top end **362**. The discharge port **364** is concentrically aligned with the air exit port **376**. In one embodiment, the base **391** of the insert **360** comprises a curved surface flush with an inner bottom surface of the reservoir **373**. The fluid channel **394** tapers from one diameter around its bottom opening to a smaller diameter at its top end **362** with the discharge port **364**. The fluid channel **394** is slightly larger in diameter than the tube **374** along the entire length of both the fluid channel **394** and the tube **374**, with the tube **374** comprising a similar conical shape having the smaller diameter on its top end. The distance between the outer surface of the tube **374** and the inner surface of the fluid channel **394** should be sufficient to create a venturi effect. When used, the discharge port **364** is the uppermost part of the irrigator **330**, with no additional barrier or structure breaking up the size or flow of the fluid drawn up from the canister **370**. In one embodiment, the distance between the outer surface of the tube **374** and the inner surface of the fluid channel **394** is about 0.0001 to about 0.010 inches (about 0.00254 - 0.254 mm)). One or more bumps **369** may be used, by way of example, to secure a tight fit and/or proper alignment between the insert **360** and the main canister **370**.

An indicator **368** (best depicted in **Figure 27**) may be used on one side of the extension **367** to assist with alignment of the flat portion **377** on the main canister **370**. The extension **367** comprises a rim **361** positioned on top of the main canister **370** and below the discharge port **364**, through which mist will pass for irrigation of a nasal passage. In one embodiment, the rim **361** engages the lip **372** of the canister **370**. In one embodiment, the extension **367** is slightly concave.

Similar to the inserts described above with extensions, the extension **367** comprises at least one groove **365** extending vertically along an exterior of the fluid channel **394** to an aperture **366** at the bottom of the fluid channel **394** or within the extension **367** adjacent to the fluid channel. The groove **365** runs vertically from a point below the discharge port **364** of the fluid channel **362** down to the aperture **366**. During use, deflected fluid will begin to flow back down the vertical groove **365**. The aperture **366** forms a channel of communication back into the reservoir **373**, which is an inner chamber formed by the mating of the canister **370** and the extension **367** of the insert **360**. As fluid exits the inner chamber, a vacuum is created which is relieved by the inflow of air and the deflected fluid into the reservoir **373** through the aperture **366**, thereby ensuring maximum usage and minimized waste of the fluid. In one embodiment, the aperture **366** in the extension **367** is located at a bottommost level of concavity of the extension **367**.

Similar to the embodiment above related to **Figure 12b**, the insert **360** has a bottom face with at least one groove (as depicted as **Figure 25B****)** forming a communication channel between the canister **370** and the insert **360**. In one embodiment, as depicted in **Figure 12b**, the bottom face of the insert **360** may also comprise a peripheral groove surrounding the bottom opening of the fluid channel to which the groove may extend from the outside edge of the bottom face. In one embodiment, the groove extends from the outside of the base to the inside of the insert. The base should comprise at least one groove but may also comprise more than one. The number of grooves as well as the width and depth of the groove will help regulate the flow of fluid up to the point that the airflow takes over the upper limit of flow. In one embodiment, the grooves may range in width from about 0.005" to about 0.150" (0.127 mm to about 3.81 mm). In one embodiment, the grooves may range in depth from about 0.001" to about 0.050" (.0254 to about 1.27 mm). In one embodiment, the base **391** of the insert comprises a curved surface flush with an inner bottom surface of the reservoir of the main canister **370**.

A cap **350** is optional but must be removed during use. When present, as shown in the assembled perspective view of **Figure 27**, the cap helps seal fluid within the irrigator to provide for storage or travel with the irrigator. The cap comprises no holes and covers the entire fluid channel **394** of the insert **360**, comprising a sealing plug (best shown in **Figure 30**) for the discharge port **364.** As with above embodiments, the cap **350** fits over the fluid channel **394** and comprises a sealing plug, in one embodiment, which projects into and fits within both the discharge port **364** and the air exit port **376** of the canister **370** to seal the reservoir **373** from the air exit port **376** and fluid channel discharge port **364** when the cap **350** is placed over the insert, as shown in the assembled view of **Figure 27**. Similar to above embodiments, the sealing plug of the cap may also seal or fit within only the discharge port **364** in other embodiments. The cap **350** may comprise an elongated conical shape in one embodiment to ensure a good fit over the insert and its apertures. Optionally, the cap **350** may comprise a flattened edge to help with alignment over the insert **360**. The bottom portion **351** of the cap mates with a portion of the top face of the extension **367** and thus its shape will depend on the curvature of the top face of the insert's extension. The cap **350** may optionally comprise one or more vertically extending indentations **352** or curved ends **353,** as best shown in **Figure 29****,** to mate with the grooves **365** and/or apertures **366**, respectively, of the insert.

Beginning with **Figure 30****,** one embodiment of an airflow regulating system within the contiguously attached pressurized air supply source **380** is depicted. Preferably, the internal components of the pressurized air supply source **380** are placed to enhance stability and feel of the irrigation device with a low center of gravity and torque generated by the motor being in the vertical axis. Such an arrangement helps engage the large muscles of the forearm for improved stability.

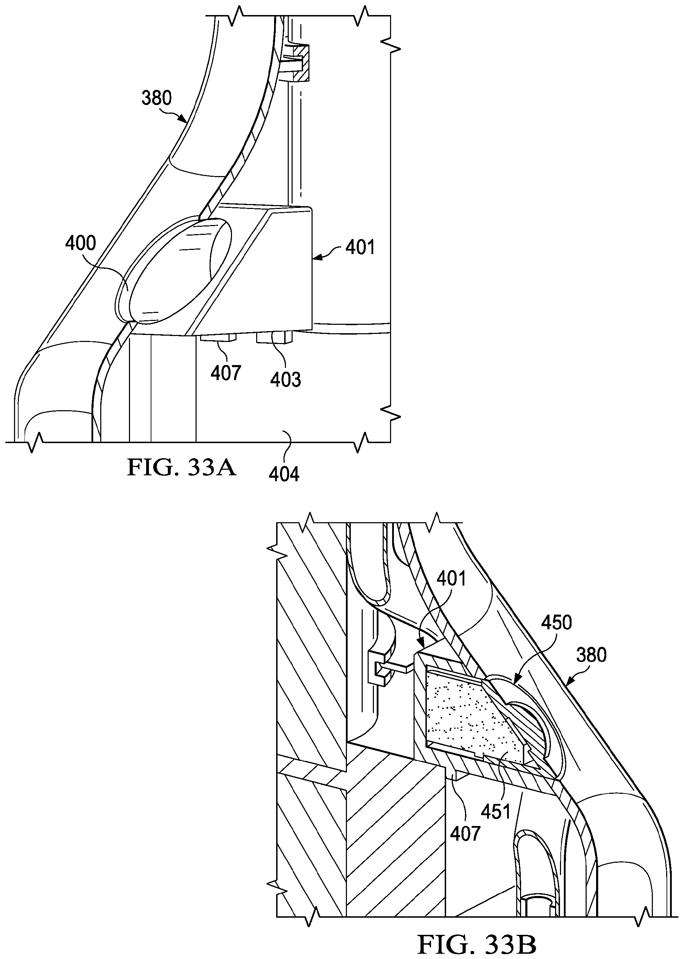

In one embodiment, the airflow regulating system comprises: a pump **386** in communication with a motor **384** and the air inlet **334** (shown in **Figure 26B**); and a filter **451** (shown in **Figure 33B**) for filtering incoming air, the filter **451** comprising an inlet air manifold **401** connected to the pump **386** and sealing against a pump air inlet post **395** of the pump **386**, as further described below. The pressurized air supply source **380** further comprises a circuit board **388**. In one embodiment, the circuit board uses a pulse-width modulation to ensure consistent motor speed. In one embodiment, the circuit board uses a programmable digital control to ensure consistent motor speed. In one embodiment, the circuit board also charges the battery from an external AC/DC converter and ensures that the motor behaves consistently whether powered by an AC/DC converter or by the battery. In one embodiment, the circuit board communicates the status of the battery to the user via an LED on the outside of the unit. In one embodiment, the motor **384** is a brushed motor. In one embodiment, the motor **384** employs caged brushes. In another embodiment, the motor **384** is a brushless motor. The outlet tubing **385**, described above, circumvents the motor **384** in one embodiment, to connect the canister **370** to a pump outlet **389** of the pump **386**. A battery **387** within the pressurized air supply source drives the motor **384** in one embodiment. In one embodiment, the battery **387** is a rechargeable lithium ion battery. In some embodiments, the battery **387** may be accessible or removable by a user. In one embodiment, the battery is not accessible to a user. In another embodiment, the motor **384** is driven by an external power supply. In one embodiment, a motor controller board utilizes pulse width modulation to control the motor speed so as to maintain a very narrow band of motor speed to regulate the airflow generated by the pump. More specifically, the motor will operate within a wide range of +/- 15% and within an operating range of +/- 4% of its set point. In one embodiment motor wires **396** are twisted to mitigate electrical noise to meet IEC-60601-1 third edition requirements for electrical emissions. In one embodiment, the motor wires **396** may also include an electronic filter that may incorporate ferrites to suppress noise. In yet another embodiment, motor wires **396** are comprised of a coaxial cable to mitigate electrical noise to suppress noise. In one embodiment, the motor **384** and the pump **386** contact the pressurized air supply source **380** through vibration dampers.

**Figures 33A and 33B** depict partial views of opposing sides of the pressurized air supply source **380**, with either half of the pressurized air supply source removed to better reflect some of its adjacent interior components; in particular to depict the air inlet manifold or **401** and filter **451** under the filter cover **450**, which resides within the opening **400** of the pressurized air supply source **380**. The air inlet manifold **401** is a small box that forms a small fitting that seals around the air inlet of the pump, eliminating the need for a tube-like communication from the outlet to the pump **386**. An opening **400** is located along the angled surface of the pressurized air supply source **380** in one embodiment. When assembled, the filter cover **450** is visible on an external side of the pressurized air supply source, within the opening **400**. In one embodiment, the filter cover **450** may be removable for access to the filter **451**.

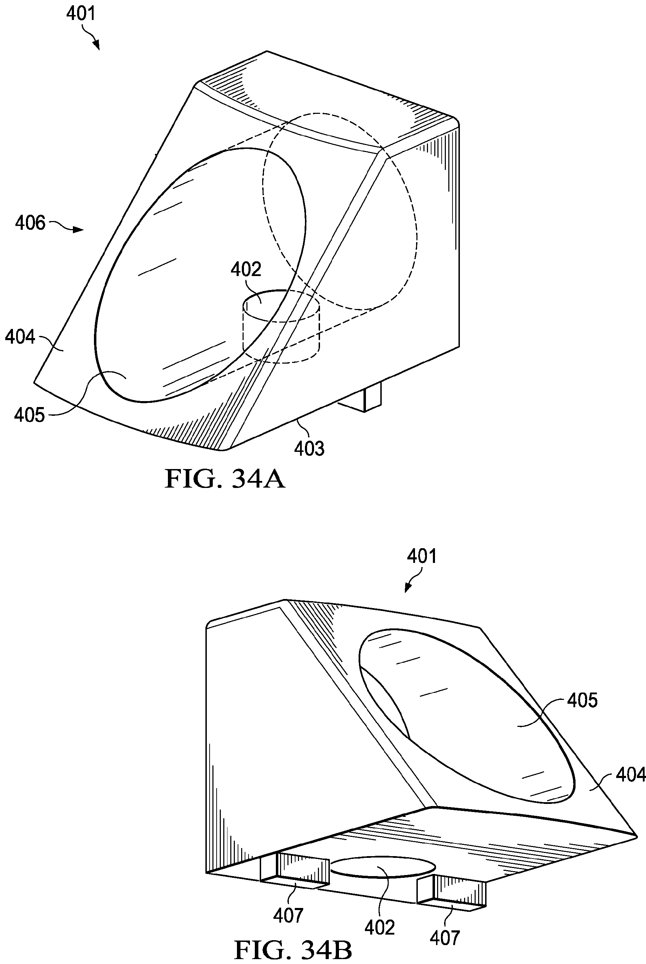

In one embodiment, the filter cover **450** is shaped to exactly match the opening **400** in the pressurized air supply source **380**. As best shown in **Figure 34A**, air inlet manifold **401** comprises a cutout **405**, which also matches the opening **400**, wherein the filter **451** is placed. The angled surface **404** of the air inlet manifold **401** matches and seals against the inside of the pressurized air supply source **380**. A cutout **402** forms an air channel within the air inlet manifold **401** to the air inlet of the pump. The cutout **402**, perhaps best shown in **Figure 34B** is at the bottom of the air inlet manifold **401** aligning with a centerline of the pump **386**. Thus, the air pathway is centered within the air inlet manifold **401**. A flat bottom **403** of the air inlet manifold **401** rests on the pump **386**. The air inlet manifold **401** thus connects and seals against a pump air inlet post **395** of the pump **386** in one embodiment. In one embodiment, the air inlet manifold **401** comprises an anti-rotation tab **407** along its bottom side to prevent movement. In one embodiment, the air inlet manifold **401** may comprise more than one anti-rotation tab **407** adjacent to the cutout **402**.

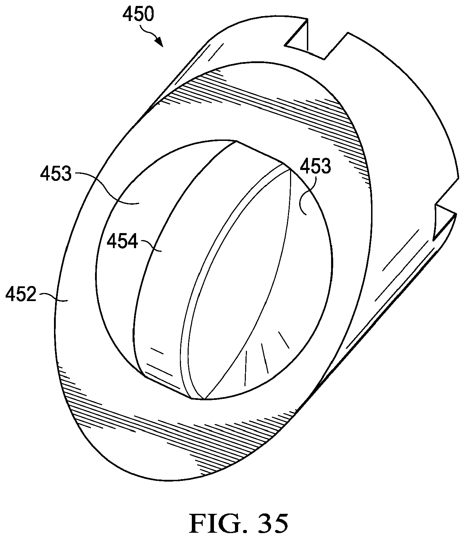

The filter cap or cover **450** is shown in more detail in **Figure 35**. The filter **451** within the air inlet manifold **401** may be composed of 80 ppi reticulated polyurethane foam in one embodiment. In one embodiment, the filter **451** is pressed and shaped within the filter cover **450**. In one embodiment, the filter **451** is accessible to a user for replacement or cleaning. Access to remove or replace the filter **451** may be accomplished by any means known in the art. A centerpiece **454** may comprise a raised, arch ridge, in one embodiment, for removal of the filter cover **450** from the manifold **401.** Face **452** is flush with the pressurized air supply source **380.** Vent **453** opens to filter **451** to allow air to pass and centerpiece **454** secures the filter inside the filter cover **450** and provides for a place for a user's fingers to insert the filter cover **450** into the manifold **401**.

As described for previous embodiments, the portable nasal irrigator **330** creates a variable particle size up to 100 microns under a pressure of 1-15 psi (0.069 - 1.0345 bar), creating a pressurized airflow that enables the resultant air-mist stream to stent-open the soft tissues of the upper airway and reach the whole nasal cavity independent of the patient's breathing. A vast majority of the particles are sized at about 20 microns. In one embodiment, the mist expelled through the exit hole comprises air and fluid particles or droplets, 100% of particles or droplets being greater than 5 microns in diameter and 99.8% of the particles or droplets are greater than 10 microns in diameter. In one embodiment, the particle or droplet diameter distribution has a mode centered around 23 microns, the mist is expelled under a pressure of 1-15 psi with a fluid delivery rate of 1-20 ml per minute, and airflow of 3-8 liters per minute, creating an air column that drives the resultant mist past the nasal valve and antrum of the nose to coat the turbinates, middle meatus to reach the posterior and superior regions of the nasal cavity and the paranasal sinus cavities without introducing the aerosol into the lungs. In one embodiment, the air pressure ranges from about 3 - 12psi (0.207 - 0.823bar), with about 1 - 12 lpm of airflow, and a fluid delivery rate of about 1 - 20ml per minute. In one embodiment, the air pressure ranges from about 4 - 8psi (0.276 - 0.552 bar), with about 3.5 - 8 lpm airflow, and about 15ml per minute fluid delivery. The resultant aerosol mist reaches the area of the nasal cavity above the inferior and posterior to the nasal turbinate or chonchae to ensure that the mist reaches the areas of the sinus ostia to clear this area of the nasal cavity and enable the natural mucociliary flow to clear the sinuses.

By way of example, a portable nasal irrigator device as described herein may be comprised of ABS, Polycarbonate, glass, stainless steel, styrolene, styrene-butadiene copolymer, co-polyester BPA-free plastics or any other plastics appropriate for medical device use, and any combination thereof. The device may further be comprised of an antimicrobial compound in some embodiments. In one embodiment, the canister and insert are constructed of a BPA-free material. In one embodiment, the canister is USP class VI compliant for the storage and delivery of drugs. In another embodiment, no latex is used in the construction of the device.

The invention illustratively disclosed herein suitably may be practiced in the absence of any element, which is not specifically disclosed herein. It should also be noted that the invention is not limited to human use, but may also be used with any number of mammals including without limitation equine, canine, feline, non-human primate, rodent, bovine, ovine, and porcine.

The description of the present invention has been presented for purposes of illustration and description, and is not intended to be exhaustive or limited to the invention in the form disclosed. Many modifications and variations will be apparent to those of ordinary skill in the art. The embodiment was chosen and described in order to best explain the principles of the invention, the practical application, and to enable others of ordinary skill in the art to understand the invention for various embodiments with various modifications as are suited to the particular use contemplated. It will be understood by one of ordinary skill in the art that numerous variations will be possible to the disclosed embodiments without going outside the scope of the invention as disclosed in the claims.

## Claims

1. A portable nasal irrigator, comprising:
(a) a main canister (370) with a reservoir (373) for holding fluid, the canister comprising at least one air exit port (376), wherein the air exit port (376) extends beyond a rim of the canister; and
(b) an insert (360) with a base (13) that fits within the canister, wherein the insert (360) comprises at least one fluid channel (394) that fits over the air exit port (376), and further wherein the fluid channel (394) is larger in diameter than the air exit port (376), thereby providing a small space between the outer surface of the air exit port (376) and the inner surface of the fluid channel (394), wherein the insert (360) comprises an extension protruding outwardly to the canister, wherein the fluid channel (394) comprises a groove extending vertically along the exterior of the fluid channel (394) to an aperture in the extension, said aperture creating a channel to the canister; and
(c) a pressurized air supply source (317) contiguously attached without tubing to the canister for introducing pressurized air through the air exit port (376) such that the fluid is expelled as a mist through an exit hole in the fluid channel (394);
a battery (387) contained within the pressurized air supply source (317); and
a filter (451) for filtering incoming air,
**characterized in that** said pressurized air supply source (317) comprises therein an airflow regulating system comprising:
an air outlet (333) comprising an air outlet elbow (382) connected to an air inlet of the main canister (370) and circumventing a motor (384) within the pressurized air supply source (317) to connect to a pump (386);
the filter (451) comprising an air inlet manifold (401) connected to the pump (386); and
a circuit board (388) to control the motor (384);
the pump (386) in communication with the main canister (370) and the motor (384), and the filter (451) connecting to the pump (386);
the motor (384) is driven by the battery (387);
the motor (384) is arranged below and adjacent the air outlet elbow (382), the pump (386) is arranged below and adjacent the motor (384), and the battery (387) is arranged below and adjacent the pump (386); and
an outlet tubing (385) connects the air outlet elbow (382) to the pump (386) circumventing the motor (384).

2. The portable nasal irrigator of claim 1 wherein the mist expelled through the exit hole comprises air and fluid particles, 100% of the fluid particles being greater than 5 microns in diameter and 99.8% of the particles or droplets are greater than 10 microns in diameter, the mist expelled under a pressure of 1-15 psi with a fluid delivery rate of 1-20 ml per minute, and an airflow of 3-8 liters per minute, thereby creating an air column that drives the mist past the nasal valve and antrum of the nose to coat a nasal cavity and paranasal sinus cavities without introducing the mist into the lungs.

3. The portable nasal irrigator of claim 1 wherein the canister is retained to and sealed together with the pressurized air supply source (317) by a mating portion (383).

4. The portable nasal irrigator of claim 1 wherein the air outlet elbow (382) comprises an angle of between 30 degrees and 90 degrees.

5. The portable nasal irrigator of claim 1 wherein the air inlet manifold (401) seals against a pump air inlet post (359) of the pump (386).

6. The portable nasal irrigator of claim 1 wherein a surface of the air inlet manifold (401) seals against the inside of the pressurized air supply source (317).

7. The portable nasal irrigator of claim 1 wherein the air outlet (333) is located within a concave opening of the pressurized air supply source (317).

8. The portable nasal irrigator of claim 1 wherein the filter (451) is housed within the air inlet manifold (401) and the air inlet manifold (401) rests on the pump (386).

9. The portable nasal irrigator of claim 1 comprising a switch for control of the irrigator.

10. The portable nasal irrigator in claim 1 wherein the canister is positively held to the pressurized air supply source (317) by a locking mechanism, the locking mechanism comprising at least one tab (378) on the canister that interfaces with a mating portion (383) that captures the tab (378) and presses the canister into a seal that seals an air channel between the canister and the pressurized air supply source (317).

11. The portable nasal irrigator in claim 1 wherein the pressurized air supply source (317) comprises an indicator light (393) source and a switch (392) integrated into a single membrane.

12. The portable nasal irrigator of claim 11 wherein the indicator light source comprises a light pipe (393) to transmit light to an external side of the pressurized air supply source (317).

13. The portable nasal irrigator of claim 1 wherein the canister is reusable or disposable.

14. The portable nasal irrigator of claim 1 comprising a cap (223) without holes therethrough, said cap substantially covering the fluid channel (394) of the insert (360) and comprising a sealing plug (233) for the discharge port (364).

15. The portable nasal irrigator of claim 1 comprising an AC/DC power supply or a rechargeable battery (387).

## Patentansprüche

1. Tragbare Nasenspülvorrichtung, umfassend:
(a) einen Hauptbehälter (370) mit einem Reservoir (373) zum Aufnehmen von Fluid, wobei der Behälter mindestens eine Luftaustrittsöffnung (376) umfasst, wobei sich die Luftaustrittsöffnung (376) über einen Rand des Behälters hinaus erstreckt; und
(b) einen Einsatz (360) mit einer Basis (13), die in den Behälter passt, wobei der Einsatz (360) mindestens einen Fluidkanal (394) umfasst, der über die Luftaustrittsöffnung (376) passt, und wobei ferner der Fluidkanal (394) im Durchmesser größer als die Luftaustrittsöffnung (376) ist, und dadurch ein kleiner Raum zwischen der äußeren Oberfläche der Luftaustrittsöffnung (376) und der inneren Oberfläche des Fluidkanals (394) bereitgestellt ist, wobei der Einsatz (360) eine Erweiterung umfasst, die nach außen zu dem Behälter vorsteht, wobei der Fluidkanal (394) eine Nut umfasst, die sich vertikal entlang der Außenseite des Fluidkanals (394) zu einer Öffnung in der Erweiterung erstreckt, wobei die Öffnung einen Kanal zu dem Behälter erzeugt; und
(c) eine Druckluftversorgungsquelle (317), die angrenzend ohne Schläuche an dem Behälter angebracht ist, um Druckluft durch die Luftaustrittsöffnung (376) einzuleiten, sodass das Fluid als Nebel durch ein Austrittsloch in dem Fluidkanal (394) ausgestoßen wird;
eine Batterie (387), die in der Druckluftversorgungsquelle (317) enthalten ist; und
einen Filter (451) zum Filtern von einströmender Luft,
**dadurch gekennzeichnet, dass** die Druckluftzufuhrquelle (317) darin ein Luftstromregulierungssystem umfasst, das Folgendes umfasst:
einen Luftauslass (333), umfassend ein Kniestück (382) des Luftauslasses, das mit einem Lufteinlass des Hauptbehälters (370) verbunden ist und einen Motor (384) in der Druckluftversorgungsquelle (317) umgeht, um mit einer Pumpe (386) verbunden zu werden;
wobei der Filter (451) einen Luftansaugrohr (401) umfasst, das mit der Pumpe (386) verbunden ist; und
eine Leiterplatte (388) zum Steuern des Motors (384);
wobei die Pumpe (386) mit dem Hauptbehälter (370) und dem Motor (384) in Verbindung steht und der Filter (451) mit der Pumpe (386) verbunden ist;
wobei der Motor (384) von der Batterie (387) angetrieben wird;
wobei der Motor (384) unter und angrenzend an das Kniestück (382) des Luftauslasses angeordnet ist, die Pumpe (386) unter und angrenzend an den Motor (384) angeordnet ist und die Batterie (387) unter und angrenzend an der Pumpe (386) angeordnet ist; und
wobei ein Auslassschlauch (385) das Kniestück (382) des Luftauslasses mit der Pumpe (386) verbindet, wodurch der Motor (384) umgangen wird.

2. Tragbare Nasenspülvorrichtung nach Anspruch 1, wobei der durch das Austrittsloch ausgestoßene Luft- und Fluidteilchen umfasst, wobei 100 % der Fluidteilchen im Durchmesser größer als 5 Mikrometer sind und 99,8 % der Teilchen oder Tröpfchen im Durchmesser größer als 10 Mikrometer sind, wobei der Sprühnebel unter einem Druck von 1 bis 15 psi mit einer Fluidabgabegeschwindigkeit von 1 bis 20 ml pro Minute und einem Luftstrom von 3 bis 8 Litern pro Minute ausgestoßen wird, wodurch eine Luftsäule erzeugt wird, welche den Sprühnebel an der Nasenklappe und dem Nasenantrum vorbei treibt, um eine Nasenhöhle und Nasennebenhöhlen zu beschichten, ohne den Sprühnebel in die Lungen einzubringen.

3. Tragbare Nasenspülvorrichtung nach Anspruch 1, wobei der Behälter an der Druckluftversorgungsquelle (317) durch einen passenden Abschnitt (383) gehalten wird und mit dieser zusammen abgedichtet ist.

4. Tragbare Nasenspülvorrichtung nach Anspruch 1, wobei das Kniestück (382) des Luftauslasses einen Winkel zwischen 30 Grad und 90 Grad umfasst.

5. Tragbare Nasenspülvorrichtung nach Anspruch 1, wobei das Luftansaugrohr (401) gegen eine Pumpen-Lufteinlasssäule (359) der Pumpe (386) abdichtet.

6. Tragbare Nasenspülvorrichtung nach Anspruch 1, wobei eine Oberfläche des Luftansaugrohrs (401) gegen die Innenseite der Druckluftversorgungsquelle (317) abdichtet.

7. Tragbare Nasenspülvorrichtung nach Anspruch 1, wobei sich der Luftauslass (333) innerhalb einer konkaven Öffnung der Druckluftversorgungsquelle (317) befindet.

8. Tragbare Nasenspülvorrichtung nach Anspruch 1, wobei der Filter (451) innerhalb des Luftansaugrohrs (401) untergebracht ist und das Luftansaugrohr (401) auf der Pumpe (386) aufliegt.

9. Tragbare Nasenspülvorrichtung nach Anspruch 1, umfassend einen Schalter zur Steuerung der Spülvorrichtung.

10. Tragbare Nasenspülvorrichtung nach Anspruch 1, wobei der Behälter durch einen Verriegelungsmechanismus formschlüssig an der Druckluftversorgungsquelle (317) gehalten wird, wobei der Verriegelungsmechanismus mindestens eine Lasche (378) an dem Behälter umfasst, die an einen passenden Abschnitt (383) anschließt, welcher die Lasche (378) hält und den Behälter in eine Dichtung drückt, die einen Luftkanal zwischen dem Behälter und der Druckluftversorgungsquelle (317) abdichtet.

11. Tragbare Nasenspülvorrichtung nach Anspruch 1, wobei die Druckluftversorgungsquelle (317) eine Signalleuchtquelle (393) und einen Schalter (392) umfasst, der in eine einzelne Membran integriert ist.

12. Tragbare Nasenspülvorrichtung nach Anspruch 11, wobei die Signalleuchtquelle eine Lichtröhre (393) umfasst, um Licht an eine äußere Seite der Druckluftversorgungsquelle (317) zu übertragen.

13. Tragbare Nasenspülvorrichtung nach Anspruch 1, wobei der Behälter wiederverwendbar oder wegwerfbar ist.

14. Tragbare Nasenspülvorrichtung nach Anspruch 1, umfassend eine Kappe (223) ohne Löcher, wobei die Kappe im Wesentlichen den Fluidkanal (394) des Einsatzes (360) abdeckt und einen Abdichtstopfen (233) für die Ausstoßöffnung (364) umfasst.

15. Tragbare Nasenspülvorrichtung nach Anspruch 1, die eine AC/DC-Stromversorgung oder eine wiederaufladbare Batterie (387) umfasst.

## Revendications

1. Irrigateur nasal portable, comprenant :
(a) une cartouche principale (370) avec un réservoir (373) pour contenir le fluide, la cartouche comprenant au moins un orifice de sortie d'air (376), dans lequel l'orifice de sortie d'air (376) s'étend au-delà d'un bord de la cartouche ; et
(b) un insert (360) avec une base (13) qui s'intègre dans la cartouche, dans lequel l'insert (360) comprend au moins un canal de fluide (394) qui s'intègre sur l'orifice de sortie d'air (376), et en outre dans lequel le canal de fluide (394) a un diamètre plus grand que l'orifice de sortie d'air (376), fournissant ainsi un petit espace entre la surface externe de l'orifice de sortie d'air (376) et la surface interne du canal de fluide (394), dans lequel l'insert (360) comprend une extension faisant saillie vers l'extérieur de la cartouche, dans lequel le canal de fluide (394) comprend une rainure s'étendant verticalement le long de l'extérieur du canal de fluide (394) jusqu'à une ouverture dans l'extension, ladite ouverture créant un canal vers la cartouche ; et
(c) une source d'alimentation en air sous pression (317) fixée de manière contiguë sans tube à la cartouche pour introduire de l'air sous pression à travers l'orifice de sortie d'air (376) de telle sorte que le fluide est expulsé sous forme de brume à travers un trou de sortie dans le canal de fluide (394) ;
une batterie (387) contenue dans la source d'alimentation en air sous pression (317) ; et
un filtre (451) pour la filtration de l'air entrant,
**caractérisé en ce que** ladite source d'alimentation en air sous pression (317) comprend à l'intérieur un système de régulation du débit d'air comprenant :
une sortie d'air (333) comprenant un coude de sortie d'air (382) raccordé à une entrée d'air de la cartouche principale (370) et contournant un moteur (384) dans la source d'alimentation en air sous pression (317) pour se raccorder à une pompe (386) ;
le filtre (451) comprenant un collecteur d'entrée d'air (401) raccordé à la pompe (386) ; et
une carte de circuit imprimé (388) pour commander le moteur (384) ;
la pompe (386) en communication avec la cartouche principale (370) et le moteur (384), et le filtre (451) se raccordant à la pompe (386) ;
le moteur (384) est entraîné par la batterie (387) ;
le moteur (384) est disposé en dessous et adjacent au coude de sortie d'air (382), la pompe (386) est disposée en dessous et adjacente au moteur (384), et la batterie (387) est disposée en dessous et adjacente à la pompe (386) ; et
un tube de sortie (385) raccorde le coude de sortie d'air (382) à la pompe (386) contournant le moteur (384).

2. Irrigateur nasal portable selon la revendication 1 dans lequel la brume expulsée à travers le trou de sortie comprend des particules d'air et de fluide, 100% des particules de fluide ayant un diamètre supérieur à 5 microns et 99,8 % des particules ou gouttelettes ont un diamètre supérieur à 10 microns, la brume expulsée sous une pression de 1 à 15 psi avec un débit de distribution de fluide de 1 à 20 ml par minute et un débit d'air de 3 à 8 litres par minute, créant ainsi une colonne d'air qui conduit la brume au-delà de la valve nasale et de l'antre du nez pour recouvrir une cavité nasale et des cavités des sinus paranasaux sans introduire la brume dans les poumons.

3. Irrigateur nasal portable selon la revendication 1 dans lequel la cartouche est retenue et scellée ensemble avec la source d'alimentation en air sous pression (317) par une portion d'accouplement (383).

4. Irrigateur nasal portable selon la revendication 1 dans lequel le coude de sortie d'air (382) comprend un angle compris entre 30 degrés et 90 degrés.

5. Irrigateur nasal portable selon la revendication 1 dans lequel le collecteur d'entrée d'air (401) est scellé contre un montant d'entrée d'air (359) de pompe de la pompe (386).

6. Irrigateur nasal portable selon la revendication 1 dans lequel une surface du collecteur d'entrée d'air (401) est scellée contre l'intérieur de la source d'alimentation en air sous pression (317).

7. Irrigateur nasal portable selon la revendication 1 dans lequel la sortie d'air (333) est située dans une ouverture concave de la source d'alimentation en air sous pression (317).

8. Irrigateur nasal portable selon la revendication 1 dans lequel le filtre (451) est logé dans le collecteur d'entrée d'air (401) et le collecteur d'entrée d'air (401) repose sur la pompe (386).

9. Irrigateur nasal portable selon la revendication 1 comprenant un interrupteur pour la commande de l'irrigateur.

10. Irrigateur nasal portable selon la revendication 1 dans lequel la cartouche est bien maintenue à la source d'alimentation en air sous pression (317) par un mécanisme de verrouillage, le mécanisme de verrouillage comprenant au moins une languette (378) sur la cartouche qui s'interface avec une portion d'accouplement (383) qui capture la languette (378) et presse la cartouche dans un joint qui scelle un canal d'air entre la cartouche et la source d'alimentation en air sous pression (317).

11. Irrigateur nasal portable selon la revendication 1 dans lequel la source d'alimentation en air sous pression (317) comprend une source de voyant lumineux (393) et un interrupteur (392) intégrés en une seule membrane.

12. Irrigateur nasal portable selon la revendication 11 dans lequel la source de voyant lumineux comprend un guide de lumière (393) pour transmettre la lumière à un côté externe de la source d'alimentation en air sous pression (317).

13. Irrigateur nasal portable selon la revendication 1, dans lequel la cartouche est réutilisable ou jetable.

14. Irrigateur nasal portable selon la revendication 1 comprenant un capuchon (223) sans trous à travers celui-ci, ledit capuchon recouvrant sensiblement le canal de fluide (394) de l'insert (360) et comprenant un bouchon d'étanchéité (233) pour l'orifice de refoulement (364).

15. Irrigateur nasal portable selon la revendication 1 comprenant une alimentation électrique AC/DC ou une batterie rechargeable (387).
